# EUROPEAN PATENT SPECIFICATION

(11) **EP 3 496 726 B1**
(45) Date of publication and mention of the grant of the patent: **28.10.2026**
(21) Application number: 17840255.8
(22) Date of filing: 10.08.2017
(51) Int. Cl.: A61K 38/17, A61P 9/10

(54) **BAG3 FOR USE IN THE TREATMENT OF ISCHEMIA-REPERFUSION INJURY**
BAG3 ZUR BEHANDLUNG VON ISCHÄMIE-REPERFUSIONSSCHÄDEN
BAG3 POUR LE TRAITEMENT DE LÉSIONS D'ISCHÉMIE-REPERFUSION

(30) Priority: 11.08.2016 US 201662373410 P
(43) Date of publication of application: 19.06.2019
(73) Proprietor: Temple University Of The Commonwealth System Of Higher Education, Philadelphia, PA 19122 (US)
(72) Inventor: FELDMAN, Arthur, M., Wynnewood, PA 19096 (US); CHEUNG, Joseph, Y., Bryn Mawr, PA 19010 (US); KHALILI, Kamel, Bala Cynwyd, PA 19004 (US)
(74) Representative: HGF
(86) International application number: PCT/US2017/046237
(87) International publication number: WO 2018/031738

(56) References cited:
- WO-A1-2010/014957
- KR-A- 20130 103 017
- YAYU MA ET AL: "Puerarin Attenuates Anoxia/Reoxygenation Injury Through Enhancing Bcl-2 Associated Athanogene 3 Expression, a Modulator of Apoptosis and Autophagy", MEDICAL SCIENCE MONITOR, vol. 22, 1 January 2016 (2016-01-01), pages 977 - 983, XP055677552, DOI: 10.12659/MSM.897379
- JIANKAI ZHANG ET AL: "Overexpression of BAG3 Attenuates Hypoxia-Induced Cardiomyocyte Apoptosis by Inducing Autophagy", CELLULAR PHYSIOLOGY AND BIOCHEMISTRY., vol. 39, no. 2, 1 January 2016 (2016-01-01), CH, pages 491 - 500, XP055677548, ISSN: 1015-8987, DOI: 10.1159/000445641
- FEIFEI SU ET AL: "Bcl-2-associated athanogene 3 protects the heart from ischemia/reperfusion injury", JCI INSIGHT, vol. 1, no. 19, 17 November 2016 (2016-11-17), XP055677543, DOI: 10.1172/jci.insight.90931

## Description

### Field of the Invention

The present invention relates to compositions for treatment and prevention of ischemia/reperfusion injury. The compositions, which can include agents that increase levels of the Bcl2-associated athanogene 3 (BAG3), can be administered to a subject suffering from ischemia/reperfusion injury or who is at risk for ischemia/reperfusion injury.

### Background

Ischemia generally refers to a restriction of blood supply to an organ or tissue. Depending upon the particular tissue the reduction in blood supply can lead to cell death and tissue damage. Paradoxically, restoration of the blood supply, also known as reperfusion, can result in additional damage to the already damaged tissue. Ischemialreperfusion injury is associated with a variety of disorders including myocardial infarction, stroke, and peripheral vascular disease. Ischemialreperfusion injury can also occur during surgery and in organs awaiting transplantation from a donor. The incidence of mortality and morbidity related to ischemia/reperfusion injury is extensive. For example, in the U.S. alone, over 735,000 individuals experience a heart attack, i.e., myocardial infarction, each year. Ischemic heart disease is the leading cause of death in the human population worldwide, with approximately 7.4 million deaths in 2012. Stroke is the second leading cause of death worldwide, with approximately 6.7 million deaths in 2012. Despite successful efforts to limit the time between the onset of coronary obstruction and coronary intervention in patients with an acute myocardial infarction, myocardial damage due to re-perfusion injury remains a major clinical problem that has failed to be influenced by multiple pharmacologic approaches. There is a continuing need for new modalities for treatment and prevention of ischemia/reperfusion injury. Yayu et al. describes puerarin attenuates anoxia/reoxygenation injury through enhancing Bcl-2 associated Athanogene 3 expression.Zhang et al. describes overexpression of BAG3 attenuates hypoxia-induced cardiomyocyte apoptosis by inducing autophagy.KR 2013 0103017 A describes a composition for preventing or treating of nenonatal hypoxia-ischemia. WO 2010/014957 A1 describes methods for treating reperfusion injuries. Feifei et al. describes Bcl-2-associated athanogene 3 protects the heart from ischemia/reperfusion injury. **Summary** The invention is defined by claim 1. Further embodiments of the invention are defined by the dependent claims In one aspect the present invention provides a pharmaceutical composition comprising a nucleic acid encoding a BAG3 polypeptide or biologically active fragment thereof, or a BAG3 polypeptide or biologically active fragment thereof for use in treating or preventing ischemia/reperfusion injury of the heart in a subject, wherein the composition is for administration in a therapeutically effective amount to increase levels of BAG3 in the heart of the subject.

Provided herein are compositions relating to the treatment of ischemia/reperfusion injury. The compositions can include administering to a subject a therapeutically effective amount of a pharmaceutical composition that increases levels of BAG3 in ischemic tissue. In some embodiments, ischemia/reperfusion injury is the result of myocardial infarction, stroke, or peripheral vascular disease. The composition can include a nucleic acid encoding a BAG3 polypeptide or fragment thereof, a BAG3 polypeptide or fragment thereof, or a proteosome inhibitor. In some embodiments, the composition is administered during reperfusion. Also provided are methods and compositions for treating a subject at risk for ischemia/reperfusion injury. In some embodiments, the subject can be scheduled for a vascular interventional procedure.

In some embodiments, the composition is administered prior to reperfusion.

### Brief Description of the Drawings

These and other features and advantages of the present invention will be more fully disclosed in, or rendered obvious by, the following detailed description of the preferred embodiment of the invention, which is to be considered together with the accompanying drawings wherein like numbers refer to like parts and further wherein:

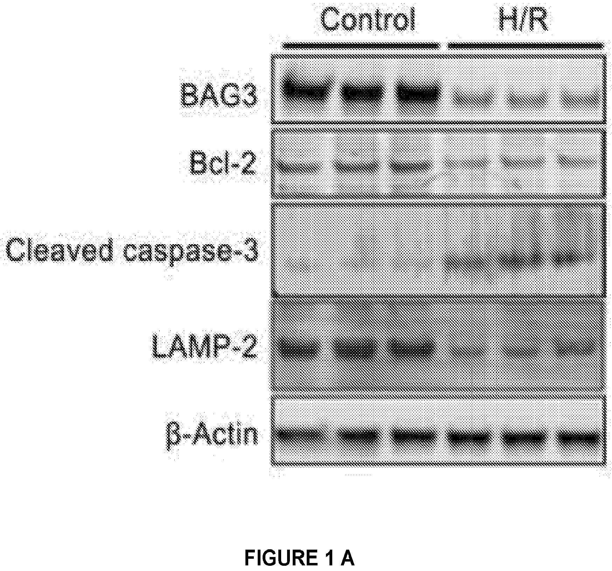

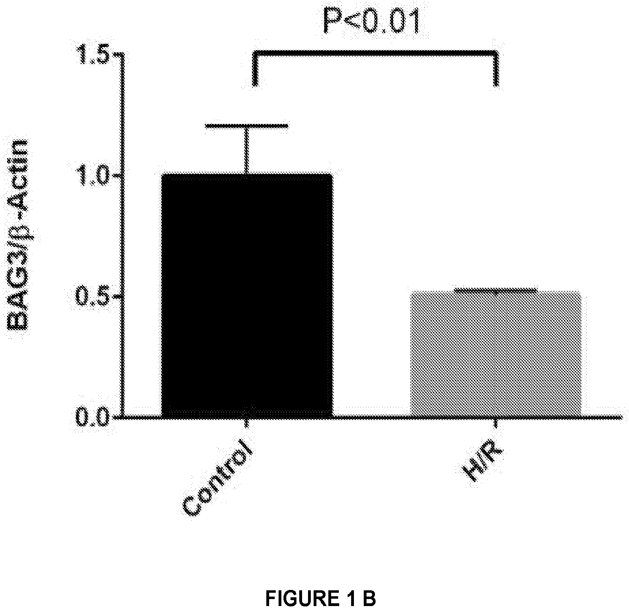

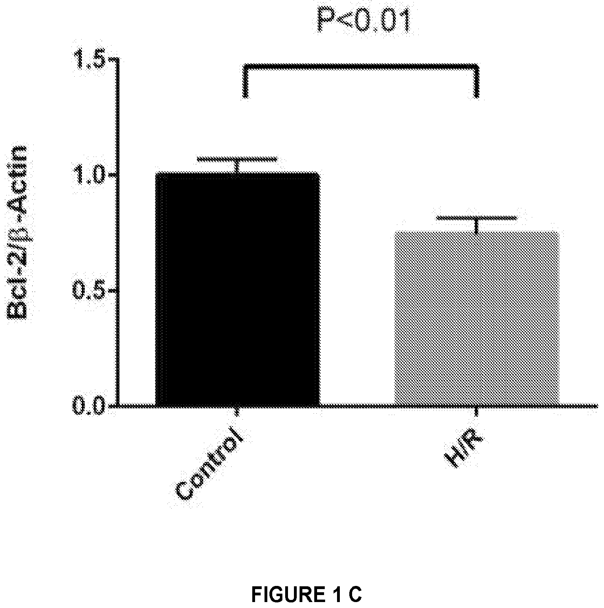

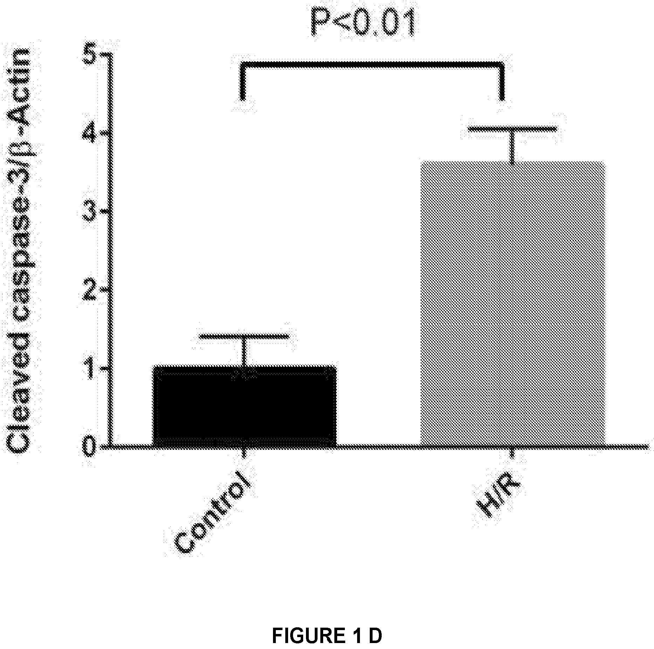

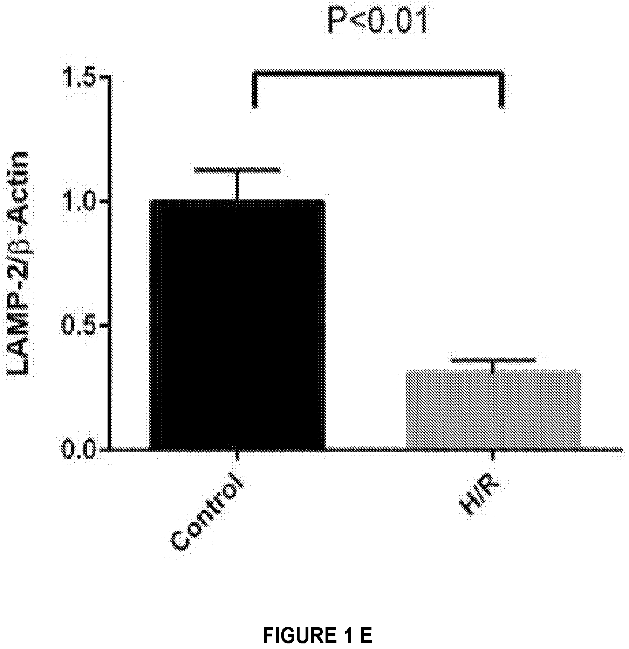

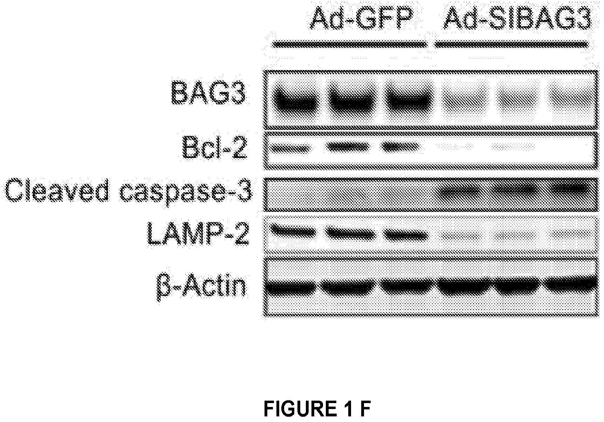

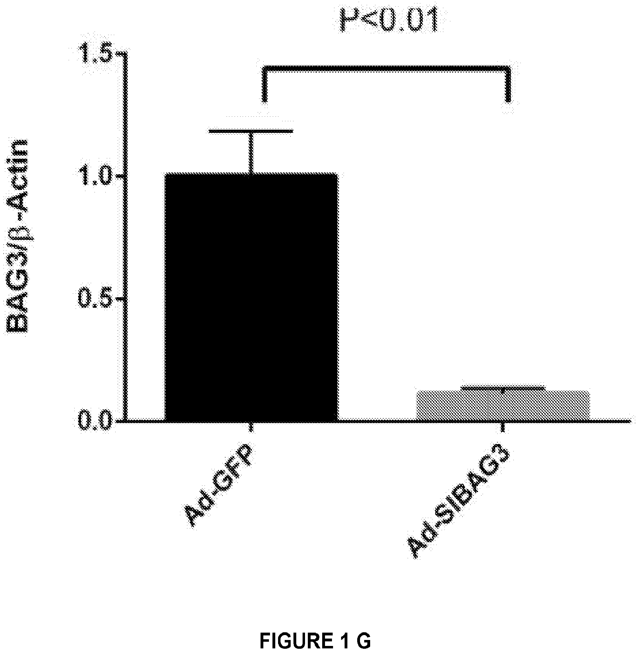

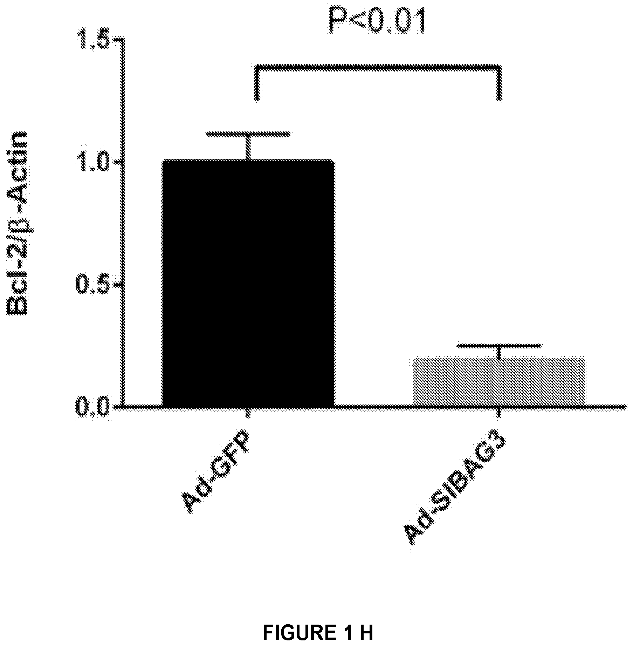

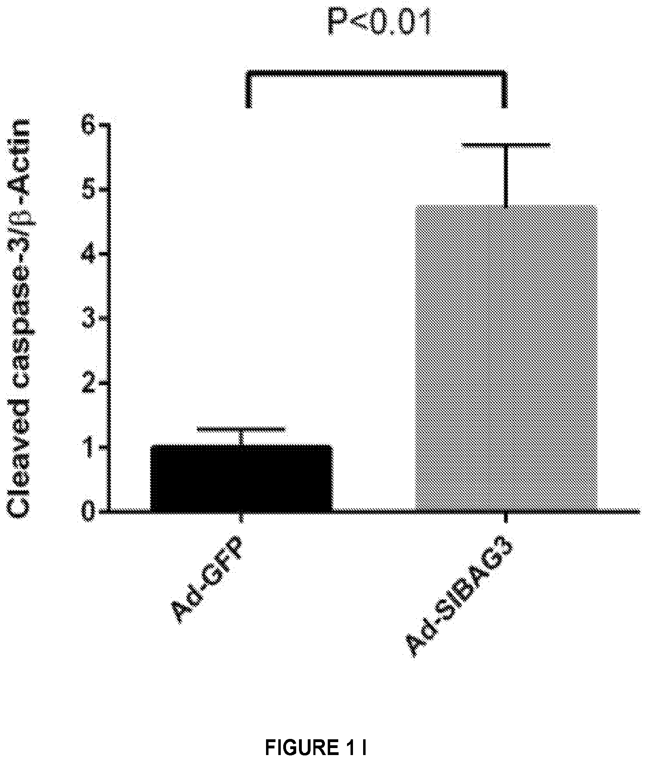

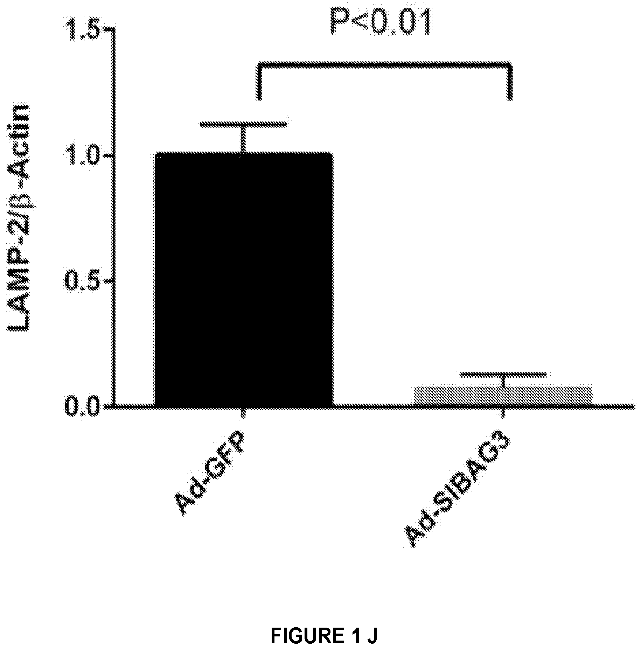

Figure 1 shows that hypoxialre-oxygenation (I/R) reduced BAG3 levels in neonatal cardiomyocytes. Neonatal mouse ventricular cardiomyocytes (NMVC) were cultured under hypoxic conditions (5% CO₂ and 95% nitrogen at 3 L/min) and in the absence of glucose for 14 hours at 37°C and then the cells were re-oxygenated for 4 hours with 5% CO₂ and 95% humidified air and with incubation medium containing glucose. Myocytes were then harvested and cellular lysates were immunoblotted for determination of levels of BAG3, cleaved-caspase-3, Bcl-2, and LAMP2. β-actin served as a control for the amount of protein loaded on the Western blot. Each experiment was repeated in three independent experiments with n=3 in each experiment. Data are presented as means ± SEM. Two-way ANOVA with Bonferroni multiple comparisons adjustments were used to assess differences across the investigational groups. Figure 1A shows a representative immunoblot of one experiment. Figure 1B shows a graph depicting the quantification of Western blots for levels of BAG3 after I/R. Figure 1C shows a graph depicting the quantification of Western blots for levels of Bcl2 after I/R. Figure 1D shows a graph depicting the quantification of Western blots for levels of cleaved caspase-3 after I/R. Figure 1E shows a graph depicting the quantification of Western blots for levels of lysosome-associated membrane protein 2 (LAMP-2) after I/R. Figure 1F shows a representative Western blot demonstrating that BAG3 knockdown by a siRNA transfected with an adenovirus (Ad-siBAG3) resulted in a significant decrease in BAG3 levels as well as a decrease in Bcl2 and LAMP-2 and an increase in cleaved caspase-3. Neonatal mouse cardiomyocytes were infected in culture with either Ad-siBAG3 or Ad-GFP (control) for 3 days after which cells were harvested and immunblotted with specific antibodies. Each experiment was repeated three times with n=3 within each individual experiment. Levels of β-actin were assessed to serve as a control for protein levels. Data presented as the mean ± SEM. Figure 1G shows a graph depicting the quantification of Western blots showing the decrease in levels of BAG3. Figure 1H shows a graph depicting the quantification of Western blots showing the decrease in levels of Bcl2. Figure 1I shows a graph depicting the quantification of Western blots showing the increase in levels of cleaved caspase3. Figure 1J shows a graph depicting the quantification of Western blots showing the decrease in levels of LAMP-2.

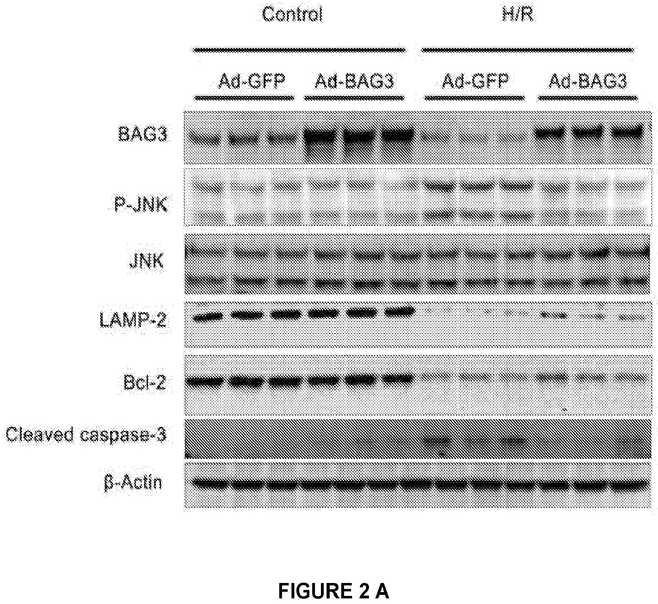

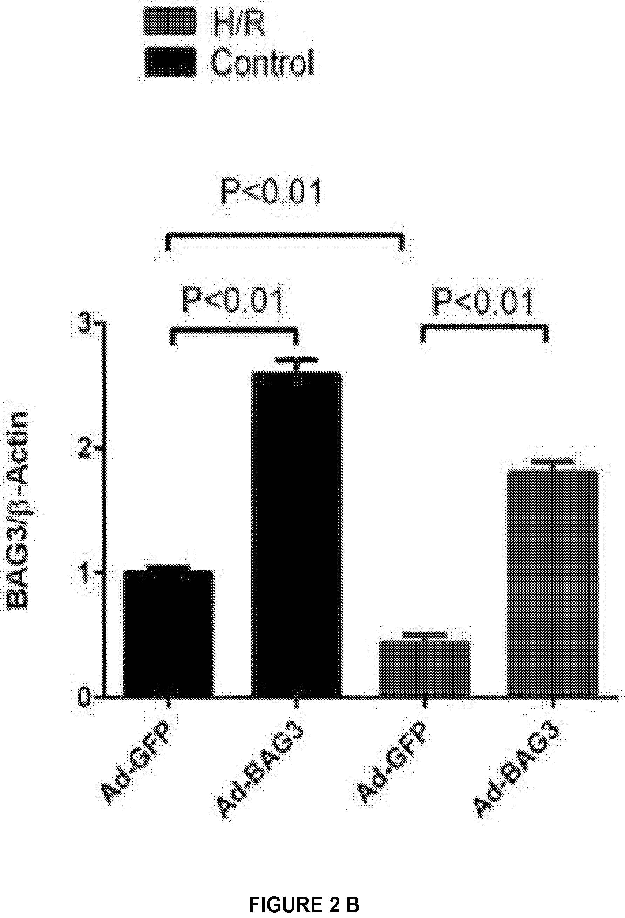

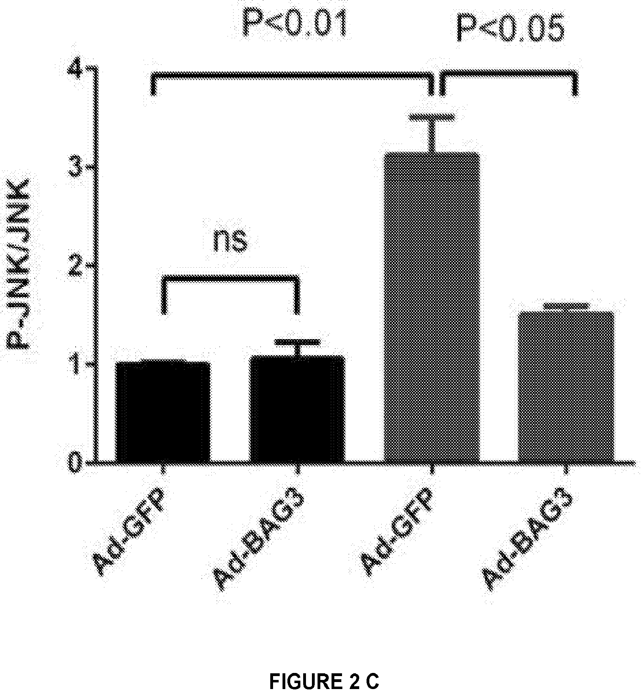

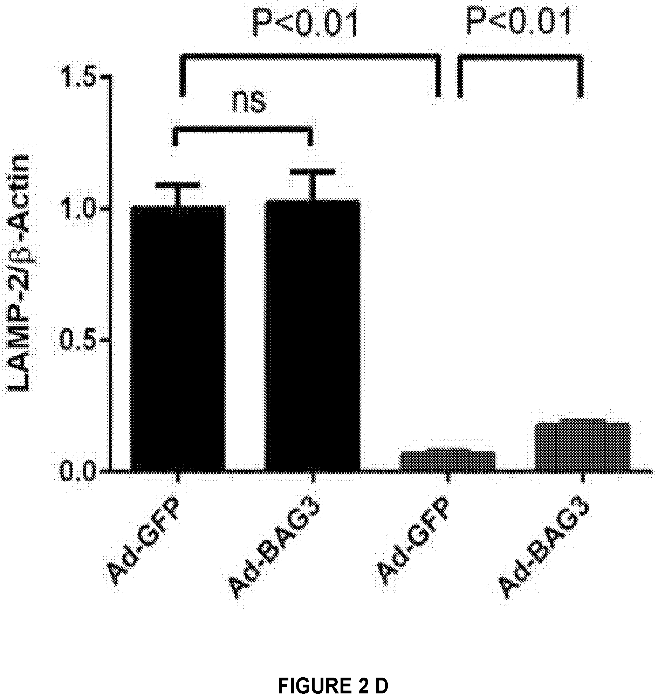

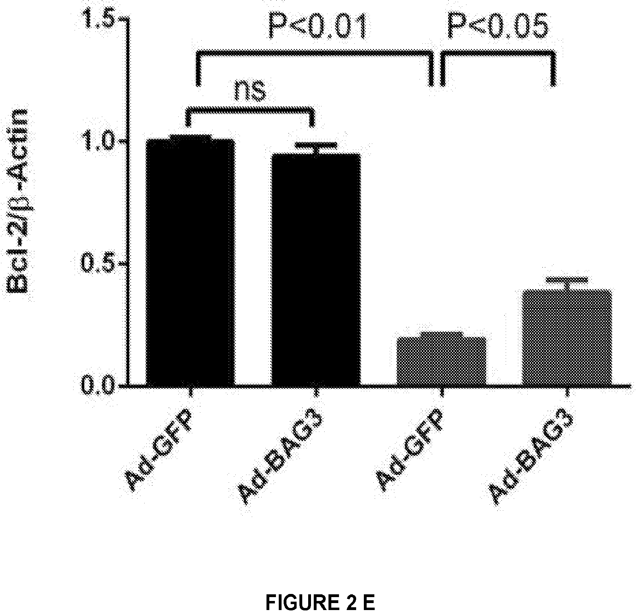

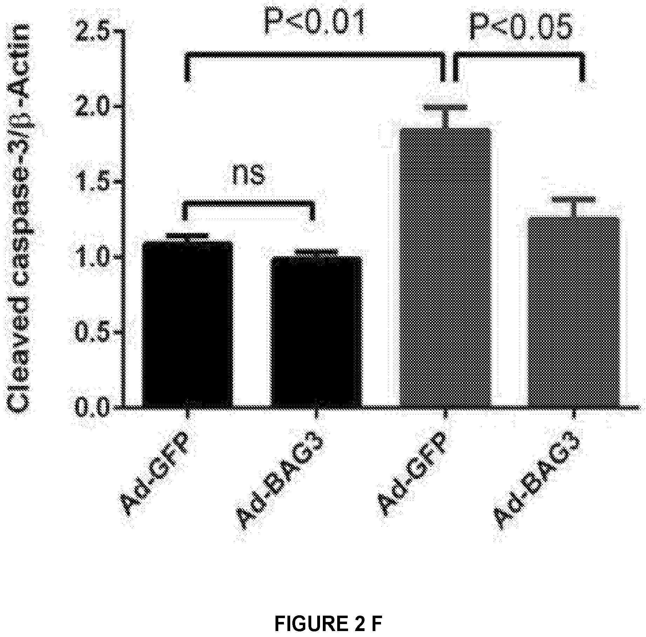

Figure 2 shows that over-expression of BAG3 ameliorated the changes in markers of apoptosis and autophagy associated with hypoxia/re-oxygenation in neonatal mouse ventricular cardiomyocytes (NMVCs) and blocked autophagy: NMVCs were infected with Ad-BAG3 or Adv-GFP for 3 days. The NMVCs were then exposed to hypoxic conditions (5% CO₂ and 95% nitrogen at 3 L/min) for 14 hours at 37°C followed by re-oxygenated for 4 hours with 5% CO₂ and 95% humidified air or were cultured under normoxic conditions (5% CO₂ and 95% humidified air). The cells were then harvested and immunoblotted. Each experiment included an n=3 for each experimental group and was repeated in three separate experiments. Figure 2A shows a representative Western blot from one of three separate experiments demonstrating that BAG3 over-expression in cells cultured under normal amounts of O₂ and CO₂ resulted in increased levels of BAG3 (depicted in the graph shown in Figure 2B) but did not significantly change levels of p-JNK (depicted in the graph shown in Figure 2C), LAMP-2 (depicted in the graph shown in Figure 2D), Bcl2 (depicted in the graph shown in Figure 2E), or cleaved caspase-3 (depicted in the graph shown in Figure 2F). By contrast, over-expression of BAG3 by Ad-BAG3 significantly altered levels of BAG3 (depicted in the graph shown in Figure 2B), p-JNK, (depicted in the graph shown in Figure 2C) LAMP-2, (depicted in the graph shown in Figure 2D) Bcl2, (depicted in the graph shown in Figure 2E) and cleaved caspase-3 (depicted in the graph shown in Figure 2F) towards levels found in NMVCs that were treated with Ad-BAG3 or Ad-GFP under normoxic conditions.

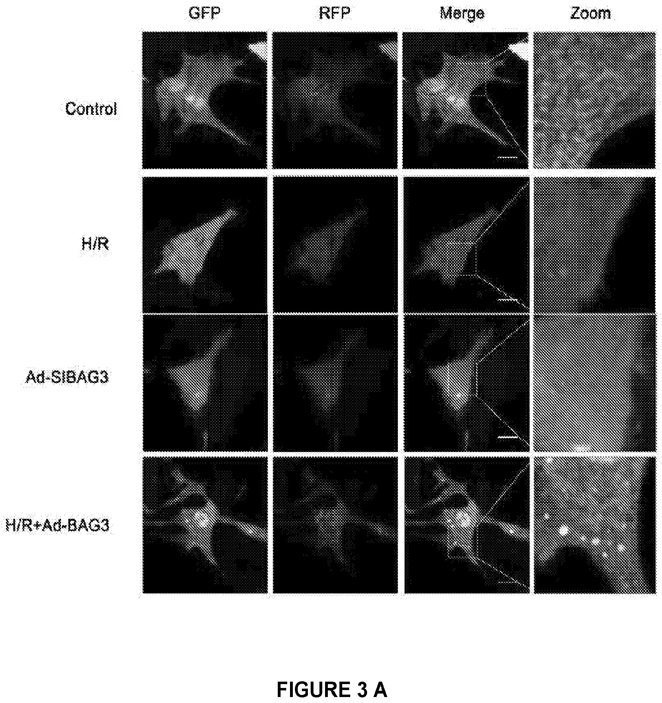

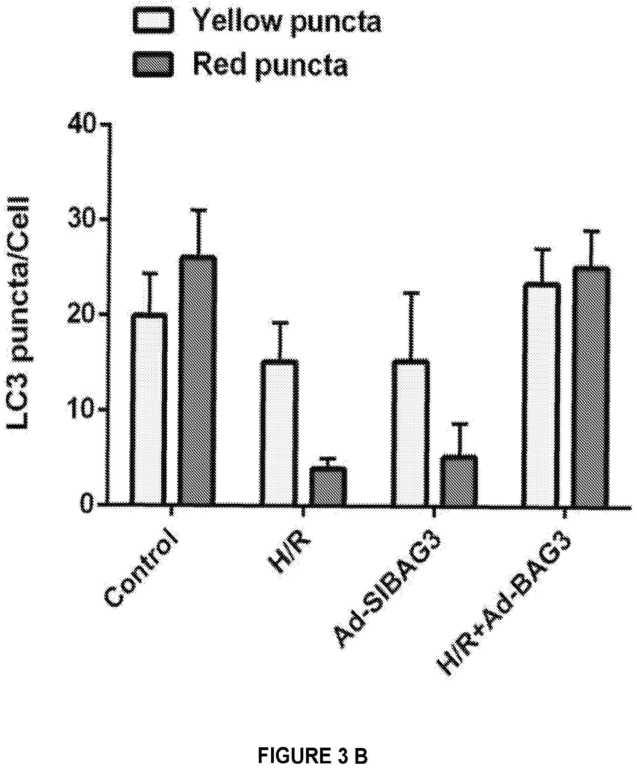

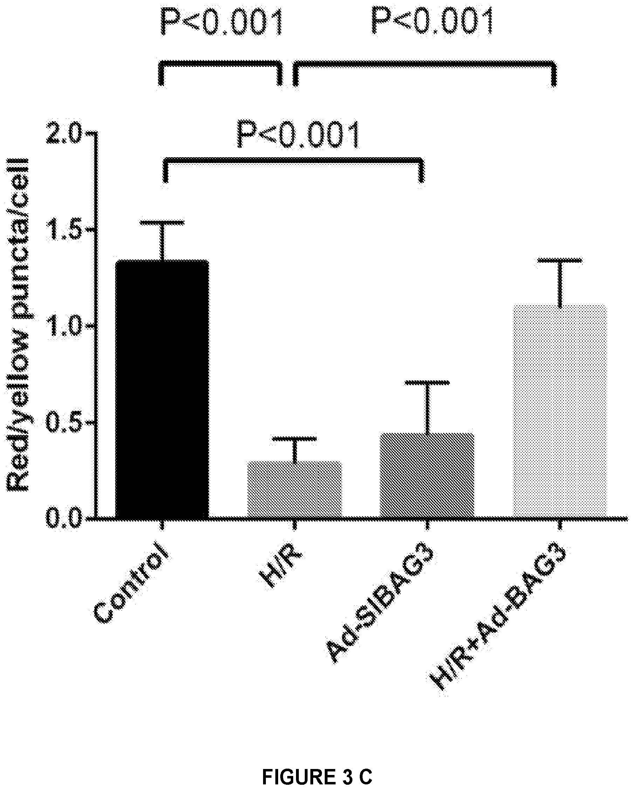

Figure 3 shows that the level of autophagy was diminished during hypoxialre-oxygenation and BAG3 knock down but was increased by over-expression of BAG3: Autophagy is not a static process but instead represents a continuing transition of phagasomes to autophagasomes and then to autolysosomes. Therefore, to determine whether autophagy was increased or decreased commensurate with changes in the cardiac levels of BAG3, NMVCs were transfected with an autophagy reporter system consisting of double-labeled mRFP-GFP-LC3-I. Both RFP (red fluorescence) and GFP (green fluorescence) could be identified in autophagasomes as yellow puncta; however, when autophagasomes fused with lysosomes, the acidity of the autolysosome quenches the GFP fluorescence resulting in predominantly red puncta. Figure 3A shows the confocal images in which yellow fluorescence was more prominent in NMVCs that had undergone H/R or that had been infected with siBAG3. By contrast, RFP signals were more prominent in cells treated with Ad-BAG3 suggesting that increased incorporation of LC3 into autolysosmes consistent with an increased level of autophagy. Figure 3B is a graph depicting a quantitative analysis of the images in Figure 3A. The subjective evaluations of the confocal images were confirmed by counting the number of yellow and red puncta in each group (control, H/R, siBAG3 and H/R + Ad-BAG3. Figure 3C is a graph comparing the ratio of autolysosomes (red puncta)/autophagasomes (yellow puncta)/total puncta in order to determine the amount of autophagy. Figure 3C shows that the ratio was significantly reduced after H/R, a change that was blunted by over-expression of BAG3 by Ad-BAG3, suggesting that both H/R and decreased levels of BAG3 blocked autophagy whereas BAG3 over-expression restored levels of autophagy.

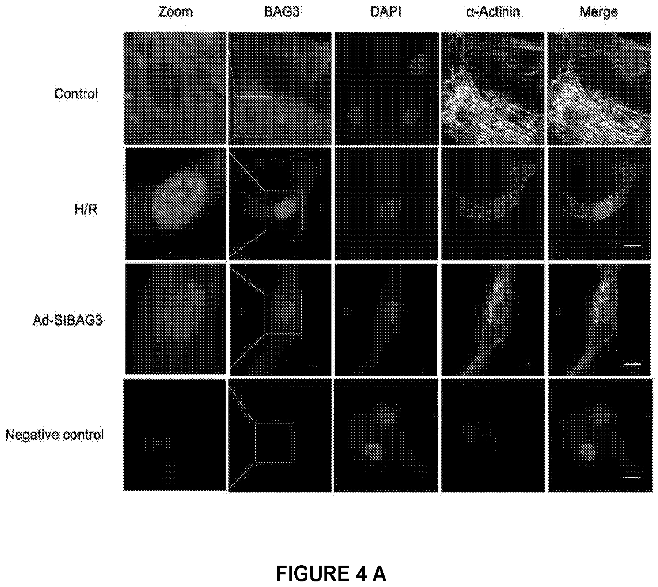

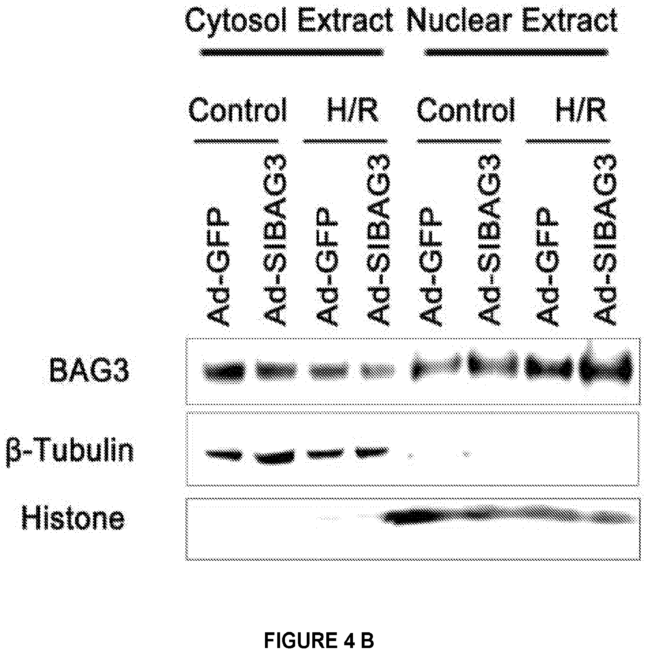

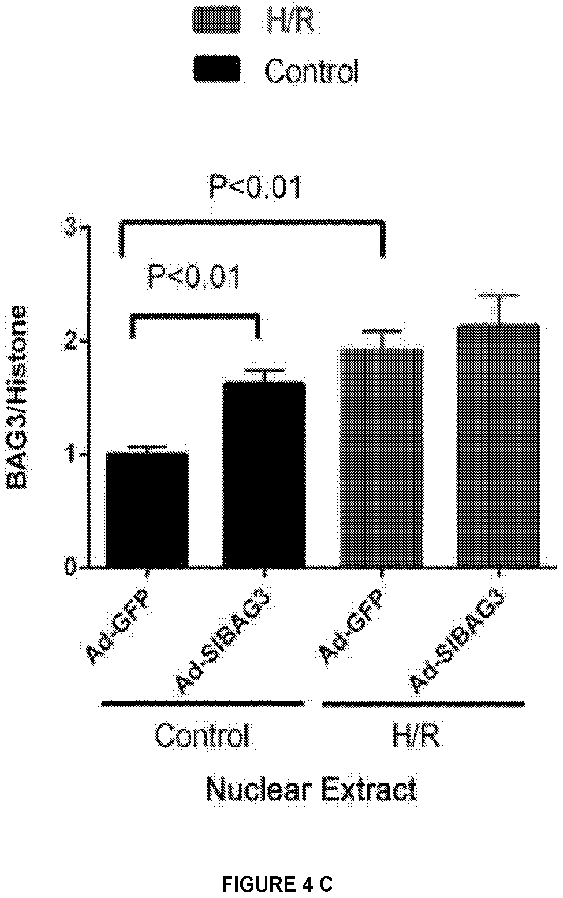

Figure 4 shows that BAG3 trans-locates to the nucleus in neonatal mouse ventricular cardiomyocytes (NMVC) after BAG3 levels are decreased by hypoxia/re-oxygenation (I/R) or after knockdown by infection with Ad-siBAG3. Figure 4A shows representative confocal images of NMVCs that underwent either H/R or BAG3 knockdown with Ad-siBAG3. NMVCs were cultured under normal conditions (CTRL), hypoxic conditions (5% CO₂ and 95% nitrogen at 3 L/min) for 14 hours at 37°C followed by re-oxygenation for 4 hours with 5% CO₂ and 95% humidified air. (HR) or were infected with Ad-siBAG3. Cardiomyocytes were then fixed and stained with either a BAG3 antibody, an α-actinin antibody or the nuclear counter-stain 4',6-Diamidino-2-Phenylindole (DAPI). A minimum of 10 cells were counted from each experiment which was repeated three times. Figure 4B shows an immunoblot analysis of BAG3 levels of cytosolic and nuclear fractions under hypoxia/re-oxygenation (I/R) or after knockdown by infection with Ad-siBAG3. NMVCs were infected with Ad-siBAG3 or Ad-GFP overnight, the media was changed and cells were incubated under normal conditions for a period of 3 days. Cells were then randomly assigned to be cultured under hypoxic conditions (5% CO₂ and 95% nitrogen at 3 L/min) for 14 hours at 37°C and then re-oxygenated for 4 hours with 5% CO₂ and 95% humidified air or to be cultured under normoxic conditions. Myocytes were then harvested and separated into cytoplasmic and nuclear fractions using stepwise lysis of cells and centrifugal isolation of nuclear and cytoplasmic protein fractions. Protein fractions were separated by SDS-Page and transferred to membranes. They were then immunoblotted with either BAG3, β-tubulin or histone antibodies. Figure 4C shows a graph depicting the quantitative analysis of three independent experiments assessing levels of BAG3 in nuclear extracts or cytoplasmic extracts of cells that were exposed to either Ad-GFP or Ad-BAG3 and then exposed to either hypoxia-re-oxygenation or to normoxic conditions. Data were expressed as the mean ± SEM and analyzed using a two-way ANOVA followed by Bonferroni correction for multiple comparisons. A p value of < 0.05 was considered significant.

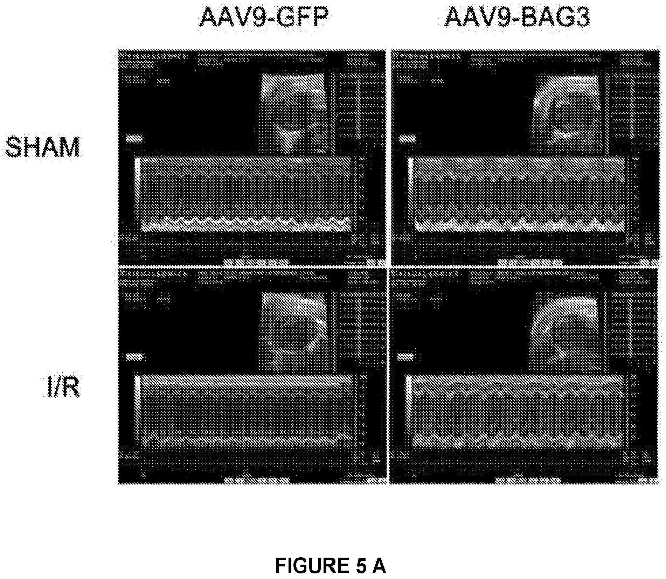

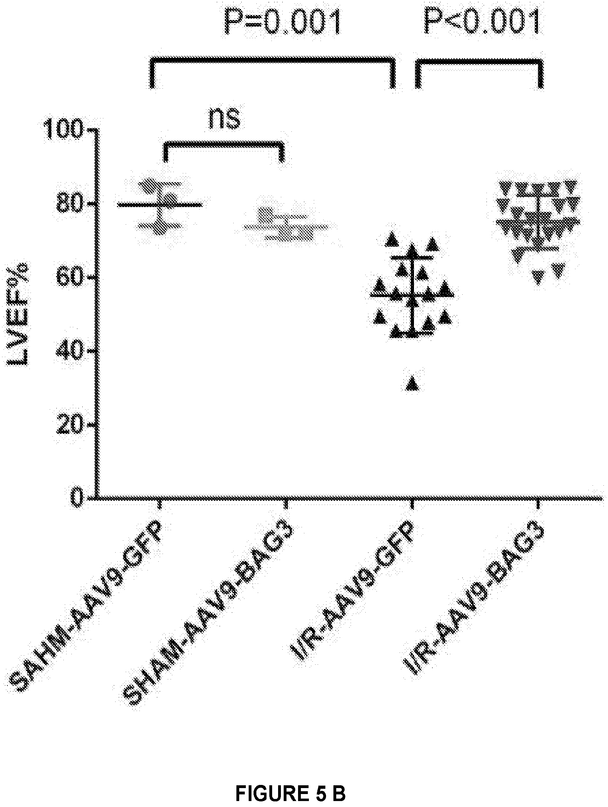

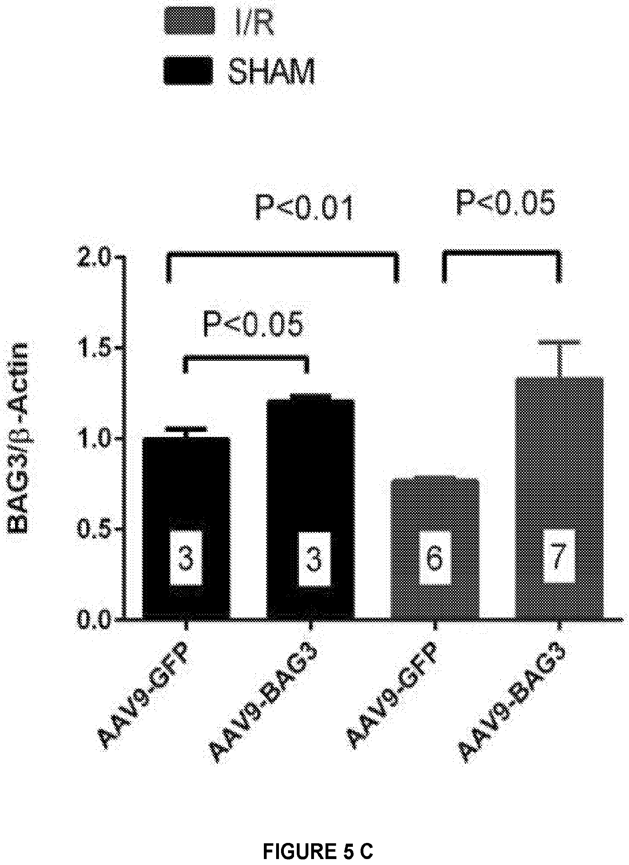

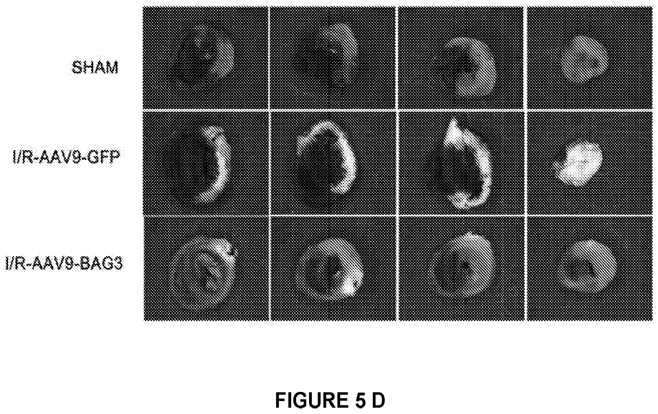

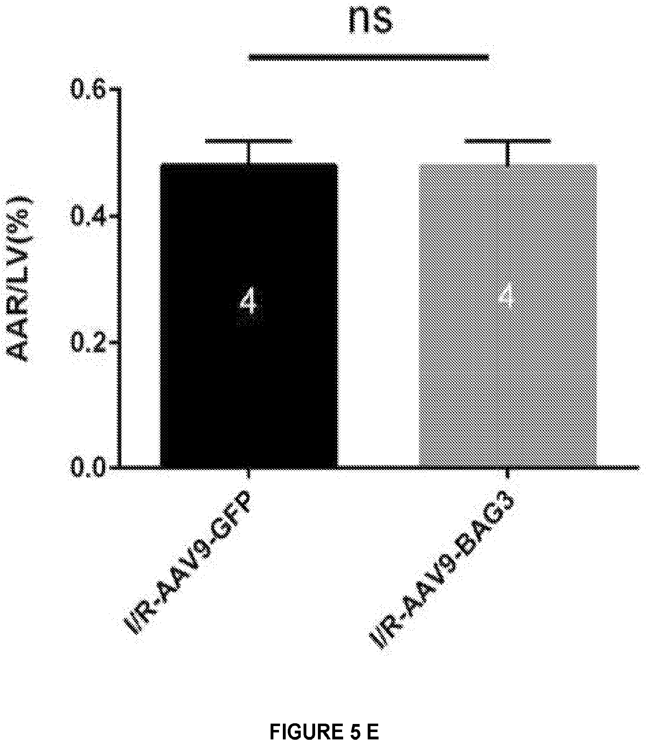

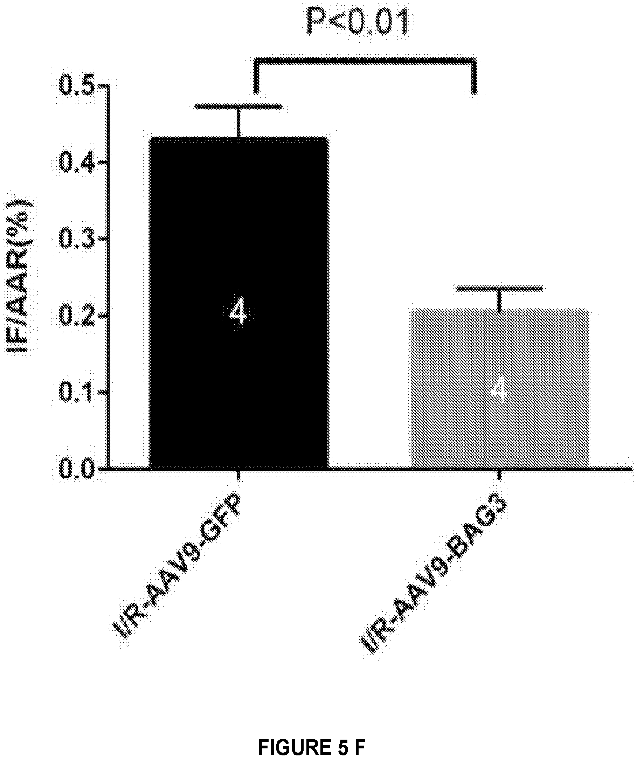

Figure 5 shows that over-expression of BAG3 preserved cardiac function and limited infarct size after ischemia/reperfusion in mice. Wild type 8 to 10 week old male FVB mice were injected via the retro-orbital plexus with adeno-associated virus sero-type 9 (AAV9) containing BAG3 under the control of the cytomegalovirus (CMV) promoter (rAAV9-BAG3). Three weeks later the left anterior descending coronary artery was occluded for 30 min followed by 72 hours of reperfusion. Cardiac function was measured by echocardiography at the conclusion of reperfusion. Figure 5A shows representative M-mode echocardiograms showing LV short axis of a wild-type FVB mouse injected with AAV9-GFP, an echo 72 hours after I/R in a mouse that was injected with AAV9-GFP and 72 hours after I/R in a mouse that was injected with AAV9-BAG3. Figure 5B shows left ventricular ejection fraction (LVEF) measured by echocardiography in mice that were injected with AAV9-BAG3 or AAV9-GFP following reperfusion for 72 hours. Figure 5C shows myocardial BAG3 levels in mice injected with AAV9-BAG3 or AAV9-GFP I/R mice after I/R. Figure 5D shows representative representative Evans Blue/triphenyltetrazolium stained cross-sections from: a sham operated mouse, a mouse that was injected with rAAV-GFP prior to I/R; and a mouse that was injected with rAAV9-BAG3 prior to I/R. The Evans Blue-stained area represents the area of the ventricle that is not at risk; the TTC-negative area represents the infarct area while the area at risk (AAR) includes both the TTC-negative area and the TTC-positive area. The area at risk (AAR) is expressed as a percent of the total LV while the area of the infarct is expressed as a percent of the AAR. Figure 5E shows quantitative assessment of area at risk (AAR/LV) in mice after AAV9-GFP or AAV9-BAG3 and subsequent I/R. Figure 5F shows infarct size in mice that received AAV9-GFP or AAV9-BAG3 three weeks prior to I/R demonstrates a significant reduction in infarct size in group receiving AAV9-BAG3.

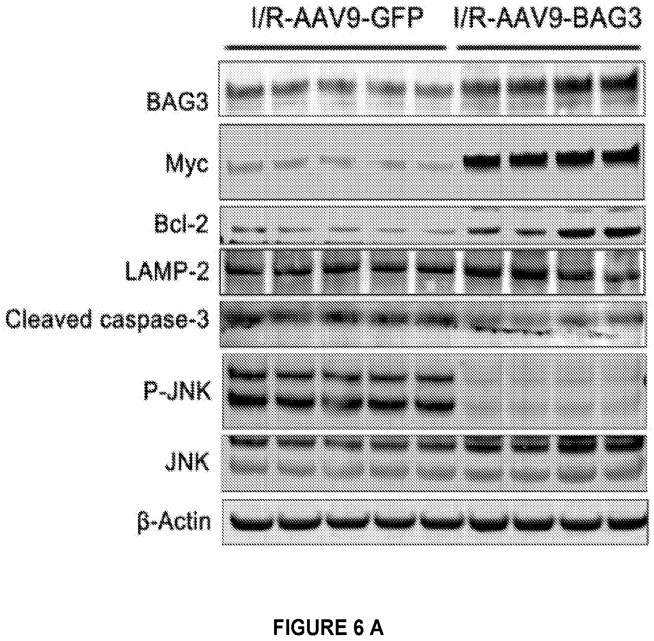

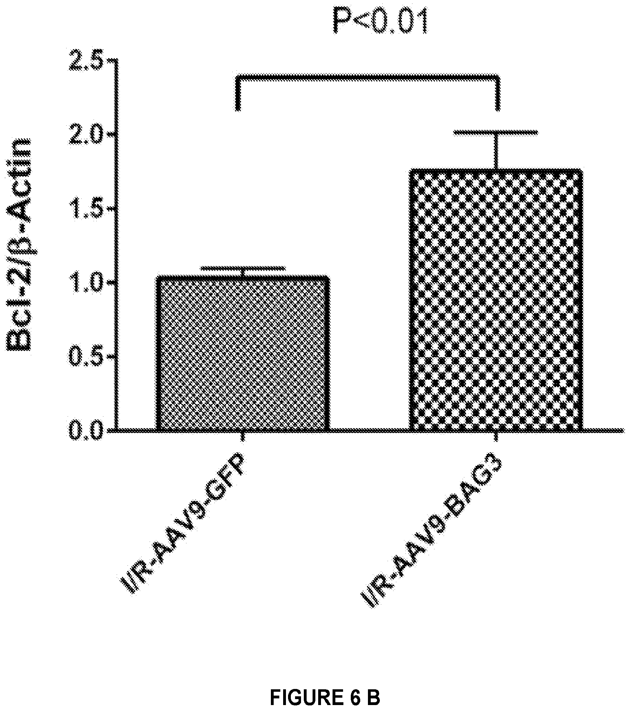

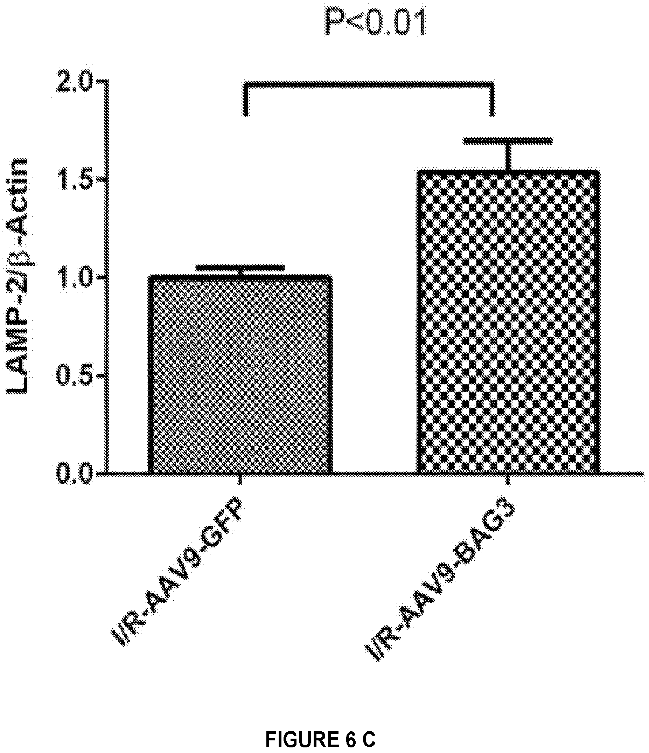

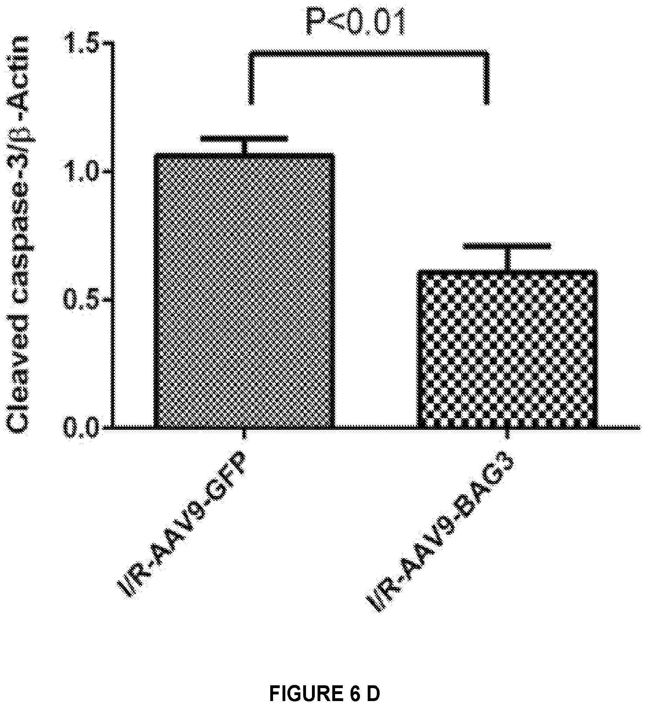

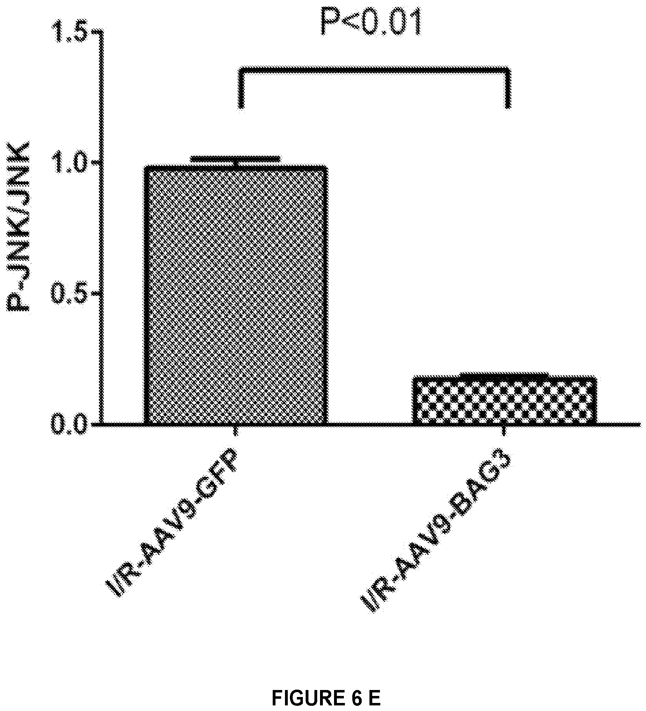

Figure 6 shows over-expression of BAG3 in mice undergoing ischemia/reperfusion ameliorates changes in markers of apoptosis and autophagy. Figure 6A shows a representative Western blot of biomarkers in tissue obtained from the border zone of wild type mice that had been injected in the retro-orbital plexus with either rAAV9-GFP or rAAV9-BAG3 three weeks prior to I/R showing changes consistent with those seen after Ad-BAG3 treatment of NMVCs. Figure 6B shows a graph depicting the quantification of Western blots (n=5 for GFP and n=4 for BAG3) of mice injected with either rAAV9-GFP or rAAV9-BAG3 prior to I/R for Bcl2. Figure 6C shows a graph depicting the quantification of Western blots (n=5 for GFP and n=4 for BAG3) of mice injected with either rAAV9-GFP or rAAV9-BAG3 prior to I/R for cleaved caspase-3. Figure 6D shows a graph depicting the quantification of Western blots (n=5 for GFP and n=4 for BAG3) of mice injected with either rAAV9-GFP or rAAV9-BAG3 prior to I/R for LAMP-2. Figure 6E shows a graph depicting the quantification of Western blots (n=5 for GFP and n=4 for BAG3) of mice injected with either rAAV9-GFP or rAAV9-BAG3 prior to I/R for p-JNK.

### Detailed Description

This description of preferred embodiments is intended to be read in connection with the accompanying drawings, which are to be considered part of the entire written description of this invention. The drawing figures are not necessarily to scale and certain features of the invention may be shown exaggerated in scale or in somewhat schematic form in the interest of clarity and conciseness. In the description, relative terms such as "horizontal," "vertical," "up," "down," "top" and "bottom" as well as derivatives thereof (e.g., "horizontally," "downwardly," "upwardly," etc.) should be construed to refer to the orientation as then described or as shown in the drawing figure under discussion. These relative terms are for convenience of description and normally are not intended to require a particular orientation. Terms including "inwardly" versus "outwardly," "longitudinal" versus "lateral" and the like are to be interpreted relative to one another or relative to an axis of elongation, or an axis or center of rotation, as appropriate. Terms concerning attachments, coupling and the like, such as "connected" and "interconnected," refer to a relationship wherein structures are secured or attached to one another either directly or indirectly through intervening structures, as well as both movable or rigid attachments or relationships, unless expressly described otherwise. The term "operatively connected" is such an attachment, coupling or connection that allows the pertinent structures to operate as intended by virtue of that relationship. When only a single machine is illustrated, the term "machine" shall also be taken to include any collection of machines that individually or jointly execute a set (or multiple sets) of instructions to perform any one or more of the methodologies discussed herein. In the claims, means-plus-function clauses, if used, are intended to cover the structures described, suggested, or rendered obvious by the written description or drawings for performing the recited function, including not only structural equivalents but also equivalent structures.

The present invention is based in part, on our finding that overexpression of Bcl2-associated athanogene3 (BAG3) protected hearts from reperfusion injury. We utilized both an *in vitro* model of hypoxia/re-oxygenation (H/R) in neonatal mouse ventricular myocytes (NMVMs) and an *in vivo* model of ischemia/reperfusion (I/R) in adult mice. We found that both hypoxial re-oxygenation (H/R) and ischemia/reperfusion (I/R) were associated with decreased levels of BAG3 and that over-expression of BAG3 in mice prior to I/R significantly reduced infarct size and improved left ventricular (LV) function.

More specifically, we found that levels of BAG3 were substantially reduced after both hypoxial re-oxygenation (H/R) in neonatal mouse ventricular cardiomyocytes (NMVCs) and ischemia/reperfusion (I/R) in the infarct border zone of the ventricular myocardium of mice. The reduced levels of BAG3 in NMVCs after H/R and in mouse heart muscle after I/R were associated with changes in the levels of markers of autophagy and/or apoptosis including increased levels of cleaved caspase 2 and decreased levels of Bcl2 and LAMP-2. We also found that BAG3 knockdown with an siRNA (siBAG3) in NMVCs resulted in an apoptosis/autophagy biomarker phenotype that exactly mirrored that seen in NMVCs after H/R. We also found that over-expression of BAG3 by an adeno-virus (Ad-BAG3) in NMVCs normalized the alterations of biomarkers for apoptosis and autophagy post-H/R. In addition, an adeno-associated virus serotype 9 coupled to BAG3 under the control of a CMV promoter (rAAV9-BAG3) significantly enhanced left ventricular (LV) function and decreased infarct size after I/R in the mouse while also modifying the levels of biomarkers for autophagy and apoptosis commensurate with that seen in NMVCs. These results suggested that normal levels of BAG3 are necessary for maintaining cardiac homeostasis during the stress of hypoxia/ischemia and reperfusion.

Accordingly, this document features compositions comprising one or more agents that increase levels of BAG3 as well as pharmaceutical formulations of agents that increase levels of of BAG3 in tissue that is at risk for or is affected by ischemial reperfusion injury. The description describes methods of administering the compositions to a patient at risk for or suffering from ischemia/reperfusion injury. The therapeutic methods described herein can be carried out in connection with other treatments, for example, drug therapies or medical devices.

### Compositions

Bcl2-associated athanogene3 (BAG3) is a 575 amino acid protein that is abundantly in the heart, skeletal muscle and in many cancers. BAG3 serves as a cochaperone with members of the heat-shock family of proteins to regulate protein quality control, interacts with Bcl2 to inhibit apoptosis, and maintains the structural integrity of the sarcomere by linking filamen with the Z-disc through binding with the actin capping protein beta-1 (CapZβ1).

BAG3 plays a role in maintaining cardiac homeostasis. Homozygous deletion of BAG3 in mice led to severe LV dysfunction, myofibril disorganization and death by four weeks of age; a single allele mutation in children was associated with progressive limb and axial muscle weakness, severe respiratory insufficiency and cardiomyopathy. Deletions in BAG3 have been associated with heart failure with reduced ejection fraction (HFrEF) independent of peripheral muscle weakness or neurologic complications; BAG3 levels were reduced in mice and pigs with HFrEF secondary to a LAD occlusion and in patients with end-stage HFrEF. Knockdown of BAG3 in neonatal myocytes led to myofibrillar disarray when the cells were stretched. In adult myocytes, BAG3 localized at the sarcolemma and t-tubules where it modulates myocyte contraction and action potential duration through specific interaction with the β1-adrenergic receptor and L-type Ca²⁺ channel.

Patients with single nucleotide polymorphisms in BAG3 and myofibrillar myopathy can have abnormalities in mitochondrial structure. We recently found that BAG3 promoted the clearance of damaged mitochondria through the autophagy-lysosome pathway and through direct interactions with mitochondria. By contrast, BAG3 knock down significantly reduced autophagy flux leading to the accumulation of damaged mitochondria and an increase in apoptosis .

BAG3, also known as MFM6; Bcl-2-Binding Protein Bis; CAIR-1; Docking Protein CAIR-1; BAG Family Molecular Chaperone Regulator 3; BAG-3; BCL2-Binding Athanogene 3; or BIS, is a cytoprotective polypeptide that competes with Hip-1 for binding to HSP 70. The NCBI reference amino acid sequence for BAG3 can be found at Genbank under accession number NP_004272.2; Public GI:14043024. We refer to the amino acid sequence of Genbank accession number NP_004272.2; Public GI:14043024 as SEQ ID NO: 1. The NCBI reference nucleic acid sequence for BAG3 can be found at Genbank under accession number NM_004281.3 GI:62530382. We refer to the nucleic acid sequence of Genbank accession number NM_004281.3 GI:62530382 as SEQ ID NO: 2. Other BAG3 amino acid sequences include, for example, without limitation, 095817.3 GI:12643665 (SEQ ID NO: 3); EAW49383.1 GI:119569768 (SEQ ID NO: 4); EAW49382.1 GI:119569767(SEQ ID NO: 5); and CAE55998.1 GI:38502170 (SEQ ID NO: 6). The BAG3 polypeptide of the invention can be a variant of a polypeptide described herein, provided it retains functionality.

This document provides agents that increase the levels of BAG3 in tissue that is at risk for or is affected by ischemial reperfusion injury. We may use the terms "increased", "increase" or "up-regulated" to generally mean an increase in the level of a BAG3 by a statistically significant amount. In some embodiments, an increase can be an increase of at least 10% as compared to a control sample or reference level, for example an increase of at least about 20%, or at least about 30%, or at least about 40%, or at least about 50%, or at least about 60%, or at least about 70%, or at least about 80%, or at least about 90% or up to and including a 100% increase or any increase between 10-100% as compared to a reference level, or at least about a 0.5-fold, or at least about a 1.0-fold, or at least about a 1.2-fold, or at least about a 1.5-fold, or at least about a 2-fold, or at least about a 3-fold, or at least about a 4-fold, or at least about a 5-fold or at least about a 10-fold increase, or any increase between 1.0-fold and 10-fold or greater as compared to a reference level.

A control sample can be a reference sample. The reference sample can be a sample obtained from the subject at one or more previous points in time. Alternatively, or in addition, a reference sample can be a standard reference level of BAG3 levels derived from a larger population of individuals. The reference population may include individuals of similar age, body size, ethnic background or general health as the subject. Thus, the levels of BAG3 can be compared to values derived from healthy individuals, i.e. individuals who are not suffering from ischemial reperfusion injury or who are not at risk for ischemial reperfusion injury. A reference sample can also be a sample obtained from a population of individuals who have recovered from ischemial reperfusion injury. The population of individuals can include individuals having a similar disorder that resulted in ischemial reperfusion injury, e.g., myocardial infarction or stroke.

### Nucleic acids

An agent that increases levels of BAG3 in a tissue that is at risk for or is affected by ischemial reperfusion injury can be a nucleic acid encoding a BAG3 polypeptide or fragment thereof. We may use the terms "nucleic acid" and "polynucleotide" interchangeably to refer to both RNA and DNA, including cDNA, genomic DNA, synthetic DNA, and DNA (or RNA) containing nucleic acid analogs, any of which may encode a polypeptide of the invention and all of which are encompassed by the invention. Polynucleotides can have essentially any three-dimensional structure. A nucleic acid can be double-stranded or single-stranded *(i.e.,* a sense strand or an antisense strand). Non-limiting examples of polynucleotides include genes, gene fragments, exons, introns, messenger RNA (mRNA) and portions thereof, transfer RNA, ribosomal RNA, siRNA, micro-RNA, ribozymes, cDNA, recombinant polynucleotides, branched polynucleotides, plasmids, vectors, isolated DNA of any sequence, isolated RNA of any sequence, nucleic acid probes, and primers, as well as nucleic acid analogs. In the context of the present invention, nucleic acids can encode a fragment of a naturally occurring BAG3 or a biologically active variant thereof.

An "isolated" nucleic acid can be, for example, a naturally-occurring DNA molecule or a fragment thereof, provided that at least one of the nucleic acid sequences normally found immediately flanking that DNA molecule in a naturally-occurring genome is removed or absent. Thus, an isolated nucleic acid includes, without limitation, a DNA molecule that exists as a separate molecule, independent of other sequences (e.g., a chemically synthesized nucleic acid, or a cDNA or genomic DNA fragment produced by the polymerase chain reaction (PCR) or restriction endonuclease treatment). An isolated nucleic acid also refers to a DNA molecule that is incorporated into a vector, an autonomously replicating plasmid, a virus, or into the genomic DNA of a prokaryote or eukaryote. In addition, an isolated nucleic acid can include an engineered nucleic acid such as a DNA molecule that is part of a hybrid or fusion nucleic acid. A nucleic acid existing among many (*e.g.,* dozens, or hundreds to millions) of other nucleic acids within, for example, cDNA libraries or genomic libraries, or gel slices containing a genomic DNA restriction digest, is not an isolated nucleic acid.

Isolated nucleic acid molecules can be produced by standard techniques. For example, polymerase chain reaction (PCR) techniques can be used to obtain an isolated nucleic acid containing a nucleotide sequence described herein, including nucleotide sequences encoding a polypeptide described herein. PCR can be used to amplify specific sequences from DNA as well as RNA, including sequences from total genomic DNA or total cellular RNA. Various PCR methods are described in, for example, PCR Primer: A Laboratory Manual, Dieffenbach and Dveksler, eds., Cold Spring Harbor Laboratory Press, 1995. Generally, sequence information from the ends of the region of interest or beyond is employed to design oligonucleotide primers that are identical or similar in sequence to opposite strands of the template to be amplified. Various PCR strategies also are available by which site-specific nucleotide sequence modifications can be introduced into a template nucleic acid.

Isolated nucleic acids also can be chemically synthesized, either as a single nucleic acid molecule (e.g., using automated DNA synthesis in the 3' to 5' direction using phosphoramidite technology) or as a series of oligonucleotides. For example, one or more pairs of long oligonucleotides *(e.g.,* >50-100 nucleotides) can be synthesized that contain the desired sequence, with each pair containing a short segment of complementarity (e.g., about 15 nucleotides) such that a duplex is formed when the oligonucleotide pair is annealed. DNA polymerase is used to extend the oligonucleotides, resulting in a single, double-stranded nucleic acid molecule per oligonucleotide pair, which then can be ligated into a vector. Isolated nucleic acids of the invention also can be obtained by mutagenesis of, *e.g.,* a naturally occurring portion of a BAG3-encoding DNA (in accordance with, for example, the formula above).

Two nucleic acids or the polypeptides they encode may be described as having a certain degree of identity to one another. For example, a BAG3 protein and a biologically active variant thereof may be described as exhibiting a certain degree of identity. Alignments may be assembled by locating short BAG3 sequences in the Protein Information Research (PIR) site (http://pir.georgetown.edu), followed by analysis with the "short nearly identical sequences" Basic Local Alignment Search Tool (BLAST) algorithm on the NCBI website (http://www.ncbi.nlm.nih.gov/blast).

As used herein, the term "percent sequence identity" refers to the degree of identity between any given query sequence and a subject sequence. For example, a naturally occurring BAG3 can be the query sequence and a fragment of a BAG3 protein can be the subject sequence. Similarly, a fragment of a BAG3 protein can be the query sequence and a biologically active variant thereof can be the subject sequence.

To determine sequence identity, a query nucleic acid or amino acid sequence can be aligned to one or more subject nucleic acid or amino acid sequences, respectively, using the computer program ClustalW (version 1.83, default parameters), which allows alignments of nucleic acid or protein sequences to be carried out across their entire length (global alignment).

ClustalW calculates the best match between a query and one or more subject sequences and aligns them so that identities, similarities and differences can be determined. Gaps of one or more residues can be inserted into a query sequence, a subject sequence, or both, to maximize sequence alignments. For fast pair wise alignment of nucleic acid sequences, the following default parameters are used: word size: 2; window size: 4; scoring method: percentage; number of top diagonals: 4; and gap penalty: 5. For multiple alignments of nucleic acid sequences, the following parameters are used: gap opening penalty: 10.0; gap extension penalty: 5.0; and weight transitions: yes. For fast pair wise alignment of protein sequences, the following parameters are used: word size: 1; window size: 5; scoring method: percentage; number of top diagonals: 5; gap penalty: 3. For multiple alignment of protein sequences, the following parameters are used: weight matrix: blosum; gap opening penalty: 10.0; gap extension penalty: 0.05; hydrophilic gaps: on; hydrophilic residues: Gly, Pro, Ser, Asn, Asp, Gln, Glu, Arg, and Lys; residue-specific gap penalties: on. The output is a sequence alignment that reflects the relationship between sequences. ClustalW can be run, for example, at the Baylor College of Medicine Search Launcher site (searchlauncher.bcm.tmc.edu/multi-align/multi-align. html) and at the European Bioinformatics Institute site on the World Wide Web (ebi.ac.uk/clustalw).

To determine a percent identity between a query sequence and a subject sequence, ClustalW divides the number of identities in the best alignment by the number of residues compared (gap positions are excluded), and multiplies the result by 100. The output is the percent identity of the subject sequence with respect to the query sequence. It is noted that the percent identity value can be rounded to the nearest tenth. For example, 78.11, 78.12, 78.13, and 78.14 are rounded down to 78.1, while 78.15, 78.16, 78.17, 78.18, and 78.19 are rounded up to 78.2.

The nucleic acids and polypeptides described herein may be referred to as "exogenous". The term "exogenous" indicates that the nucleic acid or polypeptide is part of, or encoded by, a recombinant nucleic acid construct, or is not in its natural environment. For example, an exogenous nucleic acid can be a sequence from one species introduced into another species, *i.e.,* a heterologous nucleic acid. Typically, such an exogenous nucleic acid is introduced into the other species *via* a recombinant nucleic acid construct. An exogenous nucleic acid can also be a sequence that is native to an organism and that has been reintroduced into cells of that organism. An exogenous nucleic acid that includes a native sequence can often be distinguished from the naturally occurring sequence by the presence of non-natural sequences linked to the exogenous nucleic acid, e.g., non-native regulatory sequences flanking a native sequence in a recombinant nucleic acid construct. In addition, stably transformed exogenous nucleic acids typically are integrated at positions other than the position where the native sequence is found.

Recombinant constructs are also provided herein and can be used to transform cells in order to express BAG3. A recombinant nucleic acid construct comprises a nucleic acid encoding a BAG3 sequence operably linked to a regulatory region suitable for expressing the BAG3 in the particular cell. It will be appreciated that a number of nucleic acids can encode a polypeptide having a particular amino acid sequence. The degeneracy of the genetic code is well known in the art. For many amino acids, there is more than one nucleotide triplet that serves as the codon for the amino acid. For example, codons in the coding sequence for BAG3 can be modified such that optimal expression in a particular organism is obtained, using appropriate codon bias tables for that organism.

Vectors containing nucleic acids such as those described herein also are provided. A "vector" is a replicon, such as a plasmid, phage, or cosmid, into which another DNA segment may be inserted so as to bring about the replication of the inserted segment. Generally, a vector is capable of replication when associated with the proper control elements. Suitable vector backbones include, for example, those routinely used in the art such as plasmids, viruses, artificial chromosomes, BACs, YACs, or PACs. The term "vector" includes cloning and expression vectors, as well as viral vectors and integrating vectors. An "expression vector" is a vector that includes a regulatory region. A wide variety of host/expression vector combinations may be used to express the nucleic acid sequences described herein. Suitable expression vectors include, without limitation, plasmids and viral vectors derived from, for example, bacteriophage, baculoviruses, and retroviruses.

Useful vectors include, for example, viral vectors (such as adenoviruses ("Ad"), adeno-associated viruses (AAV), lentiviruses, and vesicular stomatitis virus (VSV) and retroviruses). Replication-defective recombinant adenoviral vectors, can also be used. Vectors can also comprise other components or functionalities that further modulate gene delivery and/or gene expression, or that otherwise provide beneficial properties to the targeted cells. As described and illustrated in more detail below, such other components include, for example, components that influence binding or targeting to cells (including components that mediate cell-type or tissue-specific binding); components that influence uptake of the vector nucleic acid by the cell; components that influence localization of the polynucleotide within the cell after uptake (such as agents mediating nuclear localization); and components that influence expression of the polynucleotide. Such components also might include markers, such as detectable and/or selectable markers that can be used to detect or select for cells that have taken up and are expressing the nucleic acid delivered by the vector. Such components can be provided as a natural feature of the vector (such as the use of certain viral vectors which have components or functionalities mediating binding and uptake), or vectors can be modified to provide such functionalities. Other vectors include those described by Chen et al; BioTechniques, 34: 167-171 (2003).

A "recombinant viral vector" refers to a viral vector comprising one or more heterologous gene products or sequences. Since many viral vectors exhibit size-constraints associated with packaging, the heterologous gene products or sequences are typically introduced by replacing one or more portions of the viral genome. Such viruses may become replication-defective, requiring the deleted function(s) to be provided in trans during viral replication and encapsidation (by using, e.g., a helper virus or a packaging cell line carrying gene products necessary for replication and/or encapsidation). Modified viral vectors in which a polynucleotide to be delivered is carried on the outside of the viral particle have also been described.

Viral vectors can include a strong eukaryotic promoter operably linked to the polynucleotide e.g., a cytomegalovirus (CMV) promoter. The recombinant viral vector can include one or more of the polynucleotides therein, preferably about one polynucleotide. In some embodiments, the viral vector used in the invention methods has a pfu (plague forming units) of from about 10⁸ to about 5x 10¹⁰ pfu. In embodiments in which the polynucleotide is to be administered with a non-viral vector, use of between from about 0.1 nanograms to about 4000 micrograms will often be useful e.g., about 1 nanogram to about 100 micrograms.

Additional vectors include retroviral vectors such as Moloney murine leukemia viruses and HIV-based viruses. One HIV-based viral vector comprises at least two vectors wherein the gag and pol genes are from an HIV genome and the env gene is from another virus. DNA viral vectors include pox vectors such as orthopox or avipox vectors, herpesvirus vectors such as a herpes simplex I virus (HSV) vector.

Pox viral vectors introduce the gene into the cells cytoplasm. Avipox virus vectors result in only a short term expression of the nucleic acid. Adenovirus vectors, adeno-associated virus vectors and herpes simplex virus (HSV) vectors may be an indication for some invention embodiments. The adenovirus vector results in a shorter term expression (e.g., less than about a month) than adeno-associated virus, in some embodiments, may exhibit much longer expression. The particular vector chosen will depend upon the target cell and the condition being treated. The selection of appropriate promoters can readily be accomplished. An example of a suitable promoter is the 763-base-pair cytomegalovirus (CMV) promoter. Other suitable promoters which may be used for gene expression include, but are not limited to, the Rous sarcoma virus (RSV), the SV40 early promoter region, the herpes thymidine kinase promoter, the regulatory sequences of the metallothionein (MMT) gene, prokaryotic expression vectors such as the β-lactamase promoter, the tac promoter, promoter elements from yeast or other fungi such as the Gal 4 promoter, the ADC (alcohol dehydrogenase) promoter, PGK (phosphoglycerol kinase) promoter, alkaline phosphatase promoter; and the animal transcriptional control regions, which exhibit tissue specificity and have been utilized in transgenic animals: elastase I gene control region which is active in pancreatic acinar cells, insulin gene control region which is active in pancreatic beta cells, immunoglobulin gene control region which is active in lymphoid cells, mouse mammary tumor virus control region which is active in testicular, breast, lymphoid and mast cells, albumin gene control region which is active in liver, alpha-fetoprotein gene control region which is active in liver, alpha 1-antitrypsin gene control region which is active in the liver, beta-globin gene control region which is active in myeloid cells, myelin basic protein gene control region which is active in oligodendrocyte cells in the brain, myosin light chain-2 gene control region which is active in skeletal muscle, and gonadotropic releasing hormone gene control region which is active in the hypothalamus. Certain proteins can expressed using their native promoter. Other elements that can enhance expression can also be included such as an enhancer or a system that results in high levels of expression such as a tat gene and tar element. This cassette can then be inserted into a vector, e.g., a plasmid vector such as, pUC19, pUC118, pBR322, or other known plasmid vectors, that includes, for example, an E. *coli* origin of replication. The plasmid vector may also include a selectable marker such as the β-lactamase gene for ampicillin resistance, provided that the marker polypeptide does not adversely affect the metabolism of the organism being treated. The cassette can also be bound to a nucleic acid binding moiety in a synthetic delivery system.

In some embodiments, delivery systems can include a peripheral intravenous injection with a vector that selectively transduces only cardiomyocytes, for example, AAV serotypes that have strong cardiac tropism. Other systems involving percutaneous and surgical techniques include, for example, antegrade intra-coronary infusion either with or without coronary artery occlusion; closed-loop recirculation, wherein the vector is infused into a coronary artery removed from the circulation from the coronary sinus oxygenated extracorporeally and redeliver down the coronary artery; retrograde infusion through coronary sinus; direct myocardial injection; peripheral intravenous infusion; and pericardial injection.

In some embodiments, the polynucleotides of the invention may also be used with a microdelivery vehicle such as cationic liposomes, other lipid-containing complexes, and other macromolecular complexes capable of mediating delivery of a polynucleotide to a host cell.

Another delivery method is to use single stranded DNA producing vectors which can produce the expressed products intracellularly. See for example, Chen et al, BioTechniques, 34: 167-171 (2003) .

The vectors provided herein also can include, for example, origins of replication, scaffold attachment regions (SARs), and/or markers. A marker gene can confer a selectable phenotype on a host cell. For example, a marker can confer biocide resistance, such as resistance to an antibiotic (*e.g*., kanamycin, G418, bleomycin, or hygromycin). As noted above, an expression vector can include a tag sequence designed to facilitate manipulation or detection (*e.g*., purification or localization) of the expressed polypeptide. Tag sequences, such as green fluorescent protein (GFP), glutathione S-transferase (GST), polyhistidine, c-myc, hemagglutinin, or Flag^{™} tag (Kodak, New Haven, CT) sequences typically are expressed as a fusion with the encoded polypeptide. Such tags can be inserted anywhere within the polypeptide, including at either the carboxyl or amino terminus.

Additional expression vectors also can include, for example, segments of chromosomal, non-chromosomal and synthetic DNA sequences. Suitable vectors include derivatives of SV40 and known bacterial plasmids, e.g., *E. coli* plasmids col E1, pCR1, pBR322, pMal-C2, pET, pGEX, pMB9 and their derivatives, plasmids such as RP4; phage DNAs, e.g., the numerous derivatives of phage 1, e.g., NM989, and other phage DNA, e.g., M13 and filamentous single stranded phage DNA; yeast plasmids such as the 2µ plasmid or derivatives thereof, vectors useful in eukaryotic cells, such as vectors useful in insect or mammalian cells; vectors derived from combinations of plasmids and phage DNAs, such as plasmids that have been modified to employ phage DNA or other expression control sequences.

The vector can also include a regulatory region. The term "regulatory region" refers to nucleotide sequences that influence transcription or translation initiation and rate, and stability and/or mobility of a transcription or translation product. Regulatory regions include, without limitation, promoter sequences, enhancer sequences, response elements, protein recognition sites, inducible elements, protein binding sequences, 5' and 3' untranslated regions (UTRs), transcriptional start sites, termination sequences, polyadenylation sequences, nuclear localization signals, and introns.

As used herein, the term "operably linked" refers to positioning of a regulatory region and a sequence to be transcribed in a nucleic acid so as to influence transcription or translation of such a sequence. For example, to bring a coding sequence under the control of a promoter, the translation initiation site of the translational reading frame of the polypeptide is typically positioned between one and about fifty nucleotides downstream of the promoter. A promoter can, however, be positioned as much as about 5,000 nucleotides upstream of the translation initiation site or about 2,000 nucleotides upstream of the transcription start site. A promoter typically comprises at least a core (basal) promoter. A promoter also may include at least one control element, such as an enhancer sequence, an upstream element or an upstream activation region (UAR). The choice of promoters to be included depends upon several factors, including, but not limited to, efficiency, selectability, inducibility, desired expression level, and cell- or tissue-preferential expression. It is a routine matter for one of skill in the art to modulate the expression of a coding sequence by appropriately selecting and positioning promoters and other regulatory regions relative to the coding sequence.

### Polypeptides

In some embodiments, compositions of the invention can include a BAG3 polypeptide encoded by any of the nucleic acid sequences described above. The terms "peptide," "polypeptide," and "protein" are used interchangeably herein, although typically they refer to peptide sequences of varying sizes. We may refer to the amino acid-based compositions of the invention as "polypeptides" to convey that they are linear polymers of amino acid residues, and to help distinguish them from full-length proteins. A polypeptide of the invention can "constitute" or "include" a fragment of BAG3, and the invention encompasses polypeptides that constitute or include biologically active variants of BAG3. It will be understood that the polypeptides can therefore include only a fragment of BAG3 (or a biologically active variant thereof) but may include additional residues as well. A fragment of BAG3 and a biologically active variant of BAG3 and a fragment thereof will retain sufficient biological activity to function in the methods disclosed herein.

The bonds between the amino acid residues can be conventional peptide bonds or another covalent bond (such as an ester or ether bond), and the polypeptides can be modified by amidation, phosphorylation or glycosylation. A modification can affect the polypeptide backbone and/or one or more side chains. Chemical modifications can be naturally occurring modifications made in vivo following translation of an mRNA encoding the polypeptide (e.g., glycosylation in a bacterial host) or synthetic modifications made in vitro. A biologically active variant of BAG3 can include one or more structural modifications resulting from any combination of naturally occurring (i.e., made naturally in vivo) and with synthetic modifications (i.e., naturally occurring or non-naturally occurring modifications made in vitro). Examples of modifications include, but are not limited to, amidation (e.g., replacement of the free carboxyl group at the C-terminus by an amino group); biotinylation (e.g., acylation of lysine or other reactive amino acid residues with a biotin molecule); glycosylation (e.g., addition of a glycosyl group to either asparagines, hydroxylysine, serine or threonine residues to generate a glycoprotein or glycopeptide); acetylation (e.g., the addition of an acetyl group, typically at the N-terminus of a polypeptide); alkylation (e.g., the addition of an alkyl group); isoprenylation (e.g., the addition of an isoprenoid group); lipoylation (e.g. attachment of a lipoate moiety); and phosphorylation (e.g., addition of a phosphate group to serine, tyrosine, threonine or histidine).

One or more of the amino acid residues in a biologically active variant may be a non-naturally occurring amino acid residue. Naturally occurring amino acid residues include those naturally encoded by the genetic code as well as non-standard amino acids (e.g., amino acids having the D-configuration instead of the L-configuration). The present peptides can also include amino acid residues that are modified versions of standard residues (e.g. pyrrolysine can be used in place of lysine and selenocysteine can be used in place of cysteine). Non-naturally occurring amino acid residues are those that have not been found in nature, but that conform to the basic formula of an amino acid and can be incorporated into a peptide. These include D-alloisoleucine(2R,3S)-2-amino-3-methylpentanoic acid and L-cyclopentyl glycine (S)-2-amino-2-cyclopentyl acetic acid. For other examples, one can consult textbooks or the worldwide web (a site is currently maintained by the California Institute of Technology and displays structures of non-natural amino acids that have been successfully incorporated into functional proteins).

Alternatively, or in addition, one or more of the amino acid residues in a biologically active variant can be a naturally occurring residue that differs from the naturally occurring residue found in the corresponding position in a wildtype sequence. In other words, biologically active variants can include one or more amino acid substitutions. We may refer to a substitution, addition, or deletion of amino acid residues as a mutation of the wildtype sequence. As noted, the substitution can replace a naturally occurring amino acid residue with a non-naturally occurring residue or just a different naturally occurring residue. Further the substitution can constitute a conservative or non-conservative substitution. Conservative amino acid substitutions typically include substitutions within the following groups: glycine and alanine; valine, isoleucine, and leucine; aspartic acid and glutamic acid; asparagine, glutamine, serine and threonine; lysine, histidine and arginine; and phenylalanine and tyrosine.

The polypeptides that are biologically active variants of BAG3 can be characterized in terms of the extent to which their sequence is similar to or identical to the corresponding wild-type polypeptide. For example, the sequence of a biologically active variant can be at least or about 80% identical to corresponding residues in the wild-type polypeptide. For example, a biologically active variant of BAG3 can have an amino acid sequence with at least or about 80% sequence identity (e.g., at least or about 85%, 90%, 95%, 97%, 98%, or 99% sequence identity) to BAG3, for example, a BAG3 reference sequence such as SEQ ID NO: 2, or to a homolog or ortholog thereof.

A biologically active variant of a BAG3 polypeptide will retain sufficient biological activity to be useful in the present methods. The biologically active variants will retain sufficient activity to function in targeted DNA cleavage. The biological activity can be assessed in ways known to one of ordinary skill in the art and includes, without limitation, in vitro cleavage assays or functional assays.

Polypeptides can be generated by a variety of methods including, for example, recombinant techniques or chemical synthesis. Once generated, polypeptides can be isolated and purified to any desired extent by means well known in the art. For example, one can use lyophilization following, for example, reversed phase (preferably) or normal phase HPLC, or size exclusion or partition chromatography on polysaccharide gel media such as Sephadex G-25. The composition of the final polypeptide may be confirmed by amino acid analysis after degradation of the peptide by standard means, by amino acid sequencing, or by FAB-MS techniques. Salts, including acid salts, esters, amides, and N-acyl derivatives of an amino group of a polypeptide may be prepared using methods known in the art, and such peptides are useful in the context of the present invention.

Regardless of whether compositions are administered as nucleic acids or polypeptides, they are formulated in such a way as to promote uptake by the mammalian cell. Useful vector systems and formulations are described above. In some embodiments the vector can deliver the compositions to a specific cell type. The invention is not so limited however, and other methods of DNA delivery such as chemical transfection, using, for example calcium phosphate, DEAE dextran, liposomes, lipoplexes, surfactants, and perfluoro chemical liquids are also contemplated, as are physical delivery methods, such as electroporation, micro injection, ballistic particles, and "gene gun" systems.

In some embodiments, an agent that increases levels of BAG3 in a tissue that is at risk for or is affected by ischemial reperfusion injury can be an agent that targets the Bcl2 family. In some embodiments, the agent can be a proteosome inhibitor, for example, bortezimib.

### Methods of Treatment

The compositions disclosed herein are generally and variously useful for treatment of a subject having ischemia/ reperfusion injury or who is at risk for ischemia/reperfusion injury. We may refer to a subject, patient, or individual interchangeably.

Ischemia/reperfusion injury generally occurs following an initial ischemic insult resulting in tissue injury and/or death. Restoration of the blood supply, i.e., reperfusion, to the damaged tissue paradoxically results in further tissue damage. The underlying mechanism of reperfusion injury is not yet fully understood. In general, ischemia/reperfusion injury appears to be a complex, multifactorial phenomenon involving at least: 1) the generation of reactive oxygen species (ROS) fueled by reintroduction of molecular oxygen during reestablishment of blood flow; 2) calcium overload; 3) opening of the mitochondrial permeability transition pore (MPT), which dissipates mitochondrial membrane potential and further impairs adenosine triphosphate (ATP) production; 4) endothelial dysfunction; 5) appearance of a prothrombogenic phenotype; and 6) a pronounced inflammatory response.

Ischemia/reperfusion injury can occur in many different tissues, including the heart, brain, kidney, intestine, skeletal muscle, prostate and testis. In addition to local damage, ischemia/reperfusion can also introduce deleterious remote effects, resulting in the development of systemic inflammatory responses and multiple organ dysfunction syndrome. Most tissues can withstand short periods of ischemia that do not result in detectable injury. The length of time a specific tissue can withstand ischemia varies by cell type and organ. Typically, once a critical duration of ischemia is exceeded, cell injury and/or cell death occurs.

Ischemia in a particular tissue or organ may be caused by a loss or severe reduction in blood supply to the tissue or organ. The loss or severe reduction in blood supply may, for example, be due to coronary atherosclerosis, thromboembolic stroke, or peripheral vascular disease. Cardiac muscle ischemia is typically caused by atherosclerotic or thrombotic blockages which lead to the reduction or loss of oxygen delivery to the cardiac tissues by the cardiac arterial and capillary blood supply. Ischemia in skeletal muscle or intestinal smooth muscle may also be caused by atherosclerotic or thrombotic blockages.

Reperfusion is the restoration of blood flow to any organ or tissue in which the flow of blood had been decreased or blocked. Blood flow can be restored to an organ or tissue affected by ischemia or hypoxia. Reperfusion typically occurs as a result of a vascular interventional procedure, for example, angioplasty, for example balloon angioplasty, or a coronary artery bypass graft. Exemplary vascular interventional procedures can include procedures which employ a stent, angioplasty catheter (e.g., percutaneous transluminal angioplasty), laser catheter, atherectomy catheter, angioscopy device, beta- or gamma-radiation catheter, intravascular ultrasound device, rotational atherectomy device, radioactive balloon, heatable wire, heatable balloon, biodegradable stent strut, or biodegradable sleeve.

In some examples blood flow can be restored using a pharmaceutical agent, for example, a thrombolytic drug. In some examples blood flow can be restored using a combination of interventional procedures and pharmaceutical agents.

Symptoms of ischemia/reperfusion injury can vary depending upon the tissues or organs involved. In the case of cardiovascular tissue, ischemia/reperfusion injury can result in an increase in a extent of myocardial infarction, impaired L/V function, an increase in the severity of contractility dysfunctions, and an increase in the incidence of arrhythmia.

While we believe we understand certain events that occur upon administration of a composition that increases levels of BAG3 to a patient having or at risk for ischemia/reperfusion injury, the compositions of the present invention are not limited to those that work by affecting any particular cellular mechanism. Our working hypothesis is that increasing the levels of BAG3 in a patient having or at risk for ischemia/reperfusion injury may protect tissue from injury by maintaining cardiac homeostasis in part by limiting apoptosis and restoring autophagy.

The methods described herein are useful for the treatment of diseases or disorders that can result in ischemia/reperfusion injury or that can put a patient at risk for ischemia/reperfusion injury. Such disorders include, without limitation, myocardial infarction, heart attack, ischemic heart disease (that is, narrowing and occlusion of the coronary arteries due to plaque buildup, resulting in a reduced flow of blood and oxygen to the heart), heart failure resulting from ischemic heart disease, cardiac arrest, decreased arterial blood flow, stroke (for example, occlusion stroke), transient ischemic attack, unstable angina, cerebral vascular ischemia, peripheral vascular disease, renal failure, inflammatory disorders (e.g., rheumatoid arthritis or systemic lupus erythematosus), head trauma, drowning, sepsis, atherosclerosis, hypertension (e.g., pulmonary hypertension), drug-induced heart disease, hemorrhage, capillary leak syndrome (e.g., child and adult respiratory distress syndrome), multi-organ system failure, a state of low colloid oncotic pressure (e.g., due to starvation, anorexia nervosa, or hepatic failure with decreased production of serum proteins), anaphylaxis, hypothermia, cold injury (e.g., frostbite), hepatorenal syndrome, delirium tremens, mesenteric insufficiency, claudication, burn, electrocution, drug-induced vasodilation, drug-induced vasoconstriction, tissue rejection after transplantation, graft versus host disease, radiation exposure, a pulmonary embolus, venous thrombosis, an ischemic neurological disorder, ischemic kidney disease, or traumatic injury.

Ischemia/reperfusion injury also can result from surgery in which the blood flow and/or oxygen flow is or may be disrupted. Certain surgical procedures such as neurosurgery or cardiac surgery have a higher risk for ischemia/reperfusion injury, and even using mechanical means (e.g., a heart-lung machine) during surgery may not entirely prevent ischemia/reperfusion injury. The compositions described herein can be administered to individuals to significantly reduce or prevent ischemia/reperfusion injury that tissues and organs might experience during or following such medical emergencies (e.g., severe hypothermia or hypoxia) or procedures (e.g., surgeries).

Thus, the compositions disclosed here are useful for the treatment of a subject at risk for ischemia/reperfusion injury, for example, a patient who is about to undergo a procedure that may result in occlusion of the blood flow to the tissue during the procedure, such as a vascular interventional procedure, that is associated with ischemia/reperfusion injury. Subjects who are at increased risk for ischemia/reperfusion injury can include those at risk for a cardiovascular or ischemic event. Subjects with an increased risk of experiencing an ischemia/reperfusion injury can include, for example, smokers, diabetics, subjects with hypertension or dyslipidemia, subjects with a family history of vascular events, subjects with documented coronary disease, peripheral vascular disease, or cerebrovascular disease, or subjects undergoing diagnostic or therapeutic radiation or chemotherapy. Such subjects may also present with risk factors relating to physical inactivity, obesity, stress, alcohol use, poor diet, and age.

A subject is effectively treated whenever a clinically beneficial result ensues. This may mean, for example, a complete resolution of the symptoms of a disorder, a decrease in the severity of the symptoms of the disorder, or a slowing of the disorder's progression. These methods can further include the steps of a) identifying a subject (e.g., a patient and, more specifically, a human patient) who has or who is at risk for ischemia/reperfusion injury; and b) providing to the subject a therapeutically effective amount of a pharmaceutical composition that increases levels of BAG3. A subject can be a subject requiring a surgical procedure associated with ischemia/reperfusion injury. For example, a patient having acute myocardial infarction for whom the most effective therapeutic intervention for reducing acute myocardial ischemic injury and limiting the size of myocardial infarction is myocardial reperfusion using thrombolytic therapy or primary percutaneous coronary intervention (PPCI). A subject can be identified using standard clinical tests, for example, blood tests, chest x-rays, and electrocardiogram (ECG), an echocardiogram, a stress test, a CT scan, MRI, or cardiac catheterization. An amount of such a composition provided to the subject that results in a complete resolution of the symptoms of ischemia/reperfusion injury, a decrease in the severity of the symptoms of ischemia/reperfusion injury, or a slowing of the progression of the ischemia/reperfusion injury is considered a therapeutically effective amount. The present methods may also include a monitoring step to help optimize dosing and scheduling as well as predict outcome.

The timing of administration of the compositions can vary. In the case of acute ischemic events, for example, a heart attack, cardiopulmonary arrest, stroke, or major hemorrhagic event, the compositions can be administered prior to reperfusion. The compositions can be administered as a bolus, for example, by a first-responder (e.g., an armed services medic, an Emergency Medical Technician (EMT) or any other trained medical personnel) to the subject. Alternately or in addition to a bolus administration, the composition can be administered as a slow-drip or infusion over a period of time. For example, a slow-drip or infusion can be administered at the scene of trauma, during transport to a medical facility, and/or once the individual reaches a medical facility. Physiologically, the period immediately after injury or trauma is critical and is sometimes referred to as the "golden hour," but administration of the composition to an individual can be continued for up to 72 hours or longer (e.g., up to 1 hour, 2 hours, 4 hours, 6 hours, 8 hours, 12 hours, 18 hours, 24 hours, 36 hours, 48 hours, 60 hours, 90 hours, or more). As an alternative to a slow-drip or infusion, a bolus of a composition can be administered multiple times over, for example, a 24, 48, 72 or 90 hour period of time.

In some embodiments, the composition can be administered to a subject as soon as a potential ischemia or reperfusion injury is recognized. In some embodiments, the compositions can be administered immediately prior to or during the reperfusion treatment. The administration can continue following completion of the reperfusion treatment. In some embodiments, the compositions can be administered following the reperfusion treatment. The compositions can also be administered prior to a medical procedure that can potentially result in ischemia/reperfusion injury, for example, as part of a preoperative treatment to subject scheduled for a surgical procedure.

The methods described herein can be applied to a wide range of species, e.g., humans, non-human primates (e.g., monkeys), horses or other livestock, dogs, cats, ferrets or other mammals kept as pets, rats, mice, or other laboratory animals.

The methods of the disclosure can be expressed in terms of the preparation of a medicament. Accordingly, the disclosure encompasses the use of the agents and compositions described herein in the preparation of a medicament. The compounds described herein are useful in therapeutic compositions and regimens or for the manufacture of a medicament for use in treatment of diseases or conditions as described herein.

Any composition described herein can be administered to any part of the host's body for subsequent delivery to a target cell. A composition can be delivered to, without limitation, the heart, the brain, the cerebrospinal fluid, kidney, joints, nasal mucosa, blood, lungs, intestines, muscle tissues, skin, prostate, testis, or the peritoneal cavity of a mammal. In terms of routes of delivery, a composition can be administered by intravenous, intracranial, intraperitoneal, intramuscular, subcutaneous, intramuscular, intrarectal, intravaginal, intrathecal, intratracheal, intradermal, or transdermal injection, by oral or nasal administration, or by gradual perfusion over time. In a further example, an aerosol preparation of a composition can be given to a host by inhalation.

The dosage required will depend on the route of administration, the nature of the formulation, the nature of the patient's illness, the patient's size, weight, surface area, age, and sex, other drugs being administered, and the judgment of the attending clinicians. Wide variations in the needed dosage are to be expected in view of the variety of cellular targets and the differing efficiencies of various routes of administration. Variations in these dosage levels can be adjusted using standard empirical routines for optimization, as is well understood in the art. Administrations can be single or multiple (e.g., 2- or 3-, 4-, 6-, 8-, 10-, 20-, 50-, 100-, 150-, or more fold). Encapsulation of the compounds in a suitable delivery vehicle (e.g., polymeric microparticles or implantable devices) may increase the efficiency of delivery.

The duration of treatment with any composition provided herein can be any length of time from as short as one day to as long as the life span of the host (e.g., many years). For example, a compound can be administered once a week (for, for example, 4 weeks to many months or years); once a month (for, for example, three to twelve months or for many years); or once a year for a period of 5 years, ten years, or longer. It is also noted that the frequency of treatment can be variable. For example, the present compounds can be administered once (or twice, three times, etc.) daily, weekly, monthly, or yearly.

An effective amount of any composition provided herein can be administered to an individual in need of treatment. The term "effective" as used herein refers to any amount that induces a desired response while not inducing significant toxicity in the patient. Such an amount can be determined by assessing a patient's response after administration of a known amount of a particular composition. In addition, the level of toxicity, if any, can be determined by assessing a patient's clinical symptoms before and after administering a known amount of a particular composition. It is noted that the effective amount of a particular composition administered to a patient can be adjusted according to a desired outcome as well as the patient's response and level of toxicity. Significant toxicity can vary for each particular patient and depends on multiple factors including, without limitation, the patient's disease state, age, and tolerance to side effects.

Any method known to those in the art can be used to determine if a particular response is induced. Clinical methods that can assess the degree of a particular disease state can be used to determine if a response is induced. The particular methods used to evaluate a response will depend upon the nature of the patient's disorder, the patient's age, and sex, other drugs being administered, and the judgment of the attending clinician.

The compositions can also be administered along with other treatments. The compositions can be administered along with another therapeutic agent, for example, including, but not limited to, anti-inflammatory agents (e.g., aspirin, ibuprofen, ketoprofen, piroxicam, indomethacin, diclofenac, sulindac, naproxen, or celecoxib), vasodilators (e.g., nitroglycerin), beta blockers (e.g., alprenol, bucindolol, cartelol, carvedilol, nadolol, pindolol, propranolol, atenolol, bisoprolol, metoprolol, nebivolol, acebutolol, betaxolol, or butaxamine), cholesterol-lowering medications (e.g., statins, fibrates, nicotinic acid, bile-acid resins, or cholesterol absorption inhibitors), calcium channel blockers (e.g., lomerizine or bepridil), angiotensin-converting enzyme (ACE) inhibitors (e.g., benazepril, captopril, enalapril, fosinopril, lisinopril, moexipril, perindopril, quinapril, ramipril, or trandolapril), ranolazine, or anticoagulants (e.g., coumadins or heparins). Other exemplary agents can include adenosine, atrial natriuretic peptide, atorvastatin, cyclosporine-a, delcasertib, erythropoietin, exenatide, glucose insulin potassium (GIK) therapy, and sodium nitrate.

The compositions can also be administered along with other treatment modalities including surgery, such as a vascular interventional procedure, for example, an angioplasty, coronary artery bypass surgery, or a stent. The compositions may also be administered in conjunction with the use of a medical device. Exemplary medical devices include left ventricular assist devices.

Alternatively, or in addition, the compositions can be administered during ischemic post-conditioning (IPost), an intermittent reperfusion of acute ischemic myocardium. Other treatment realities include, without limitation, remote ischemic conditioning, therapeutic hypothermia, and therapeutic hyperoxemia.

The compositions can also be administered in conjunction with lifestyle modifications such as smoking cessation, weight loss, physical exercise, diet control, and a reduction in alcohol intake.

Concurrent administration of two or more therapeutic agents does not require that the agents be administered at the same time or by the same route, as long as there is an overlap in the time period during which the agents are exerting their therapeutic effect. Simultaneous or sequential administration is contemplated, as is administration on different days or weeks. The therapeutic agents may be administered under a metronomic regimen, e.g., continuous low-doses of a therapeutic agent.

Dosage, toxicity and therapeutic efficacy of such compositions can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀.

The data obtained from the cell culture assays and animal studies can be used in formulating a range of dosage for use in humans. The dosage of such compositions lies preferably within a range of circulating concentrations that include the ED₅₀ with little or no toxicity. The dosage may vary within this range depending upon the dosage form employed and the route of administration utilized. For any composition used in the method of the invention, the therapeutically effective dose can be estimated initially from cell culture assays. A dose may be formulated in animal models to achieve a circulating plasma concentration range that includes the IC₅₀ (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

### Articles of manufacture

The compositions described herein can be packaged in suitable containers labeled, for example, for use as a therapy to treat a subject having a suffering from or at risk for ischemia/reperfusion injury. The containers can include a composition comprising an agent that increases the levels of BAG3 in ischemic tissue.

**In** some examples , the agent can be nucleic acid sequence encoding a BAG3 polypeptide or fragment thereof or a vector encoding that nucleic acid, and one or more of a suitable stabilizer, carrier molecule, flavoring, and/or the like, as appropriate for the intended use. In some examples , the agent can be BAG3 polypeptide or fragment thereof, and one or more of a suitable stabilizer, carrier molecule, flavoring, and/or the like, as appropriate for the intended use. In some examples , the agent can be an agent that increases BAG3 expression or activity in the targeted tissue, and one or more of a suitable stabilizer, carrier molecule, flavoring, and/or the like, as appropriate for the intended use. In some examples , the agent can be a proteosome inhibitor, for example, bortezimib. Accordingly, packaged products (e.g., sterile containers containing one or more of the compositions described herein and packaged for storage, shipment, or sale at concentrated or ready-to-use concentrations) and kits, including at least one composition of the invention, e.g., a nucleic acid sequence encoding a BAG3 polypeptide or fragment thereof or a vector encoding that nucleic acid. A product can include a container (e.g., a vial, jar, bottle, bag, or the like) containing one or more compositions of the disclosure . In addition, an article of manufacture further may include, for example, packaging materials, instructions for use, syringes, delivery devices, buffers or other control reagents for treating or monitoring the condition for which prophylaxis or treatment is required.

**In** some examples , the kits can include one or more additional therapeutic agents. The additional agents can be packaged together in the same container the agent that increases the levels of BAG3 in ischemic tissue, that is, a nucleic acid sequence encoding a BAG3 polypeptide or fragment thereof or a vector encoding that nucleic acid, BAG3 polypeptide or fragment thereof, or a produce some inhibitor, or they can be packaged separately. The agent that increases the levels of BAG3 in ischemic tissue and the additional agent may be combined just before use or administered separately.

The product may also include a legend (e.g., a printed label or insert or other medium describing the product's use (e.g., an audio- or videotape)). The legend can be associated with the container (e.g., affixed to the container) and can describe the manner in which the compositions therein should be administered (e.g., the frequency and route of administration), indications therefor, and other uses. The compositions can be ready for administration (e.g., present in dose-appropriate units), and may include one or more additional pharmaceutically acceptable adjuvants, carriers or other diluents and/or an additional therapeutic agent. Alternatively, the compositions can be provided in a concentrated form with a diluent and instructions for dilution.

### Examples

### Example 1: materials and methods

Animal Protocols: Neonatal mice were obtained from female FVB mice within three days of birth (Jackson Laboratory, Bar Harbor, ME). Eight- to ten-week old male FVB mice (Jackson Laboratory) were used for assessment of infarct size after 30 min of coronary ligation and subsequent reperfusion as described previously. Sham-operated control animals were treated in an identical manner except that the LAD was not ligated. All experiments were performed according to the National Institutes of Health *Guide for the Care and Use of Laboratory Animals* and were approved by the Temple University Institutional Animal Care and Use Committee (ACUP#4031).

**Preparation of primary neonatal mouse ventricular myocyte (NMVC):** NMVCs were isolated from 1 to 3 days old FVB mice using a Pierce Primary Cardiomyocyte Isolation Kit (Cat.88281, Thermo scientific, Rockford, IL) according to manufacturer's instructions. Myocytes were seeded into each well of 6 well plates at a concentration of 2 x 10⁶ cells per plate and cultured in Dulbecco's modified Eagle's medium (DMEM, GIBCO, CA) with 1% fetal bovine serum (Denville Scientific Inc. Holliston, MA) and 1% penicillinstreptomycin (ThermoFisher Scientific, Waltham, MA). After 24 hours, the complete medium was replaced with fresh DMEM containing Cardiomyocyte Growth Supplement at 37 °C and 5% CO₂.

**Construction and use of BAG3 adenovirus:** Adenovirus expressing either GFP (Ad-GFP), BAG3 (Ad-BAG3) or siBAG3 (Ad-siBAG3) was constructed using the BD Adeno-X Expression System 2PT3674-1 and BD knockout RNAi Systems PT3739 (BD Biosciences-Clontech, Palo Alto, CA) as previously described. Forty-eight hours after isolation, NMVCs were infected with adenovirus at a multiplicity of infection of 8. NMVCs were exposed to adenovirus overnight after which media was aspirated and fresh media was applied. Media was changed daily. Experiments were then performed 72 hours after infection.

**HypoxialRe-oxygenation:** NMVCs were subjected to H/R as described previously with modifications. In brief, NMVCs were exposed to humidified 5% CO₂: 95% N₂ for 14 hours at 37 °C and incubated in glucose free medium. Cells were then re-oxygenated with 5% CO₂:95% humidified air for 4 hours in medium containing glucose. Medium was replaced daily.

**Immunoblotting:** Hearts were excised and left ventricles separated into infarct border (3 mm of proximal most end of apex) and remote zones (proximal septum). Tissues were quickly frozen in liquid nitrogen and stored at -80° C until use. Membrane proteins were prepared as described previously. In brief, tissue was lysed in buffer (Cell Signaling Technologies, Beverly, MA) containing protease and phosphatase inhibitor cocktail (ThermoScientific; Rockford, IL) and homogenized with beads in a Bullet Blender (Next Advance, Averill Park, NY). NMVCs were rinsed with ice-cold PBS, collected and lysed in buffer. After centrifugation at 13,000g for 5 min at 4°C, the supernatant was collected and protein level determined by Bradford assay (Bio-Rad, Philadelphia, PA). Equal amounts of protein (90 µl) were mixed with 30µl of 4X NuPAGE SDS sample buffer (ThermoFisher, Carlsbad, CA, USA) and 15ul of 10X NuPAGE reducing agent (ThermoFisher,), boiled, separated on NuPAGE Novex 4-12% Bis-Tris Protein Gels (ThermoFisher) using NuPAGE electrophoresis system (ThermoFisher), and transferred to nitrocellulose membranes (LiCor, Lincoln, NE). Membranes were blocked in Odyssey blocking buffer (LiCor) for 1 hour at room temperature before incubation with primary antibodies overnight. The membranes were washed with 1X PBS-T (0.1% Tween 20) and incubated with secondary antibody for 1 hour at room temperature. Protein band signals were detected with an Odyssey scanner. Primary antibodies were Myc (Cell Signaling Technologies), BAG3 (Protein Tech), Bcl-2 (Cell Signaling Technologies), LAMP-2 (ThermoFisher), cleaved Caspase-3 (Cell Signaling Technologies), JNK (Santa Cruz Biotechnology, Dallas, TX), phospho-JNK (Cell Signaling Technologies), histone, β-Tubulin, and β-actin (Santa Cruz Biotechnology). Secondary antibodies were: goat anti-mouse IRDye 800 (LiCor) and IRDye 680 goat anti-rabbit (Rockland, Gilbertsville, PA).

**Confocal Microscopy:** Confocal microscopy was used to detect BAG3 localization in adult cardiomyocytes as described previously. Briefly, neonatal mouse LV cardiomyocytes were isolated and plated on laminin-coated 4-well chamber slides (Lab-Tek., Rochester, NY). BAG3 was identified using a primary rabbit antibody (1:200; Proteintech Group Inc, Chicago IL) and α-sarcomeric actinin was identified using a mouse antibody (1:200, Sigma Ldrich). The secondary antibody was Alexfluor 594-labeled goat anti-rabbit antibodies (1:500 Invitrogen, Eugene, OR) and mounting media contained 4',6-diamidino-2-phenylindole dihydrochloride (DAPI)(Vector Laboratories Burlingame, CA). A Carl Zeiss 710 confocal microscope (63× oil objective) with ZEN software was used for imaging for BAG3 (594 nm ex., 667 nm em.), α-actinin (488 nm ex., 543 nm em.) and DAPI (405 nm ex., 495 nm em.). Total laser intensity and photomultiplier gain were set constant for all groups and settings and data were verified by two independent observers who were blinded to the experimental group. A minimum of three coverslips were used for each experimental group and at least three cell images were acquired from each coverslip.

**Autophagy RFP-GFP-LC3 reporter** system: Isolated NMVCs were infected with an adenovirus expressing mRFP-GFP-LC3 at multiplicity of infection of one as described previously . NMVCs were subjected to H/R 24 hrs after infection and then fixed with paraformaldehyde in phosphate buffered saline. After rinsing with PBS, the cells were permeabilized with 0.3% Triton X-100 in 10% normal goat serum blocking solution (Invitogen, Life technologies corporation, Frederick. MD) for 60 min. Coverslips were mounted to slides with Hardset anti-fade mounting medium (Vector Laboratories., Burlingame, Ca) and confocal imaging was performed as described above with mRFP acquired at 594 nm excitation and 667 nm emission and GFP acquired at 488 nm excitation and 543 nm emission. The puncta of seven to 10 cells in each experimental group were counted after obtaining digital images. The number of yellow puncta in the merged channel represented the number of autophagasomes. The number of autolysosomes (autophagosome-lysosome fusion) were represented by the number of red puncta as described previously .

**Cell fractionation:** The cytoplasm and nuclear fractions of NMVCs were prepared using a NE-PER nuclear and cytoplasmic extraction reagent kit (Thermo scientific, Rockford, IL, USA) according to manufacturer's instructions. Both the cytoplasmic and nuclear extractions were stored at -80°C until use for western blot.

**Construction and administration of rAAV9-BAG3:** A sequence encoding the murine myc-tagged BAG3 (NCBI accession #BC145765) was inserted into a pAAV vector that contained a cytomegalovirus (CMV) promoter. (Vector Biolabs, Malvern, PA) The construct was then packaged into AAV-9 by transfection of HEK293 cells, and viral particles were purified by CsCl₂ centrifugation (Vector Biolabs). Recombinant AAV9-BAG3 also expressed green fluorescent protein (GFP); however, GFP was not in sequence with BAG3. Fidelity of the clone and the final vector were confirmed by sequencing. Both MI mice and Sham mice were randomly assigned to receive either 60-80 µl rAAV9-BAG3 (5.0-6.5 X 10¹³ genome copies (GC)/ml) or rAAV9-GFP control (3.1 x 10¹² GC/ml) in sterile PBS at 37°C by injection into the retro-orbital venous plexus as described previously.

**Echocardiography:** Global LV function was evaluated in all mice after light sedation (2% isoflurane) using a VisualSonics Vevo 770 imaging system and a 707 scan head (Miami, FL) as described previously. The left ventricular ejection fraction (LVEF) was calculated using the formula EF% = [(LVEDV - LVESV)/LVEDV]×100; where LVEDV and LVESV are left ventricular end-diastolic volume and left ventricular end-systolic volume, respectively.

**Determination of infarct** size: Myocardium was stained with 2% triphenyltetrazolium (TTC) to measure infarct size as previously described. In brief, 72 h after I/R, the slipknot around the LAD was retied followed by injection of 2% Evans Blue dye (0.2 ml). Hearts were excised, and LV was sliced into three 1.2 mm thick slices perpendicular to the short axis of the heart and incubated in PBS containing TTC. After 20 min. at room temperature, the slices were digitally photographed. The Evans Blue-stained area (area not at risk), TTC-negative area (infarcted myocardium) and area at risk (AAR; includes both TTC-negative and positive areas) were measured with computer-based image analyzer SigmaScan Pro 5.0 (SPSS Science, Chicago, IL). AAR was expressed as percent of total LV while infarcted myocardium was expressed as percent of AAR. For Western blot analysis, the border zone included the area of the ventricle 3 mm from the apex of the heart.

**Statistical Analysis:** Data were analyzed using Graph Pad Prizm 6 or JMP version 12. Data are presented as means ± SEM for continuous variables. Two-way ANOVA with Bonferroni multiple comparisons adjustments were used to assess differences across the investigational groups. For Western blot analysis, a p-value of p<0.05 was considered significant. The control for each experiment (e.g., Ad-GFP or normoxia) was set as 1.0).

### Example 2: Hypoxic/reoxygenation decreases BAG3 levels in NMVCs

BAG3 levels were significantly decreased in NMVCs after H/R (Figure 1A and B; p<0.01) when compared to normoxic controls. NMVCs were prepared and cultured according to the methods in Example 1. Briefly, NMVCs were cultured under hypoxic conditions (5% CO₂ and 95% nitrogen at 3 L/min) and in the absence of glucose for 14 hours at 37°C and then the cells were re-oxygenated for 4 hours with 5% CO₂ and 95% humidified air and with incubation medium containing glucose. To explore potential signaling pathways by which reduced BAG3 levels post-H/R might influence cell injury, we measured markers of apoptosis and autophagy. Myocytes were harvested and cellular lysates were immunoblotted for determination of levels of BAG3, cleaved-caspase-3, Bcl-2, and LAMP2. β-actin served as a control for the amount of protein loaded on the Western blot. Each experiment was repeated in three independent experiments with n=3 in each experiment. As shown in Figure 1, levels of Bcl-2 (Figure 1C; p<0.01) and LAMP-2 (Figure 1E; p<0.01) were significantly decreased, while levels of cleaved caspase-3 (Figure 1D; p<0.01) were significantly increased when compared to normoxic controls.

To assess whether the reduction in BAG3 levels alone was sufficient to altering the levels of markers of apoptosis and autophagy, we reduced endogenous BAG3 in NMVCs using an siRNA (Ad-siBAG3) by approximately 90% when compared with cells infected with Ad-GFP control (Figure 1F and G). NMVCs were infected in culture with either Ad-siBAG3 or Ad-GFP (control) for 3 days as described in Example 1 after which cells were harvested and immunblotted with specific antibodies. Changes in markers of apoptosis and autophagy observed in NMVCs post-H/R were recapitulated in NMVCs in which BAG3 expression was reduced by siRNA as levels of cleaved caspase-3 were increased (Figure 1H; p<0.01) while levels of Bcl2 (Figure 1I; p<0.01) and LAMP-2 (Figure 1J; p<0.01) were significantly reduced as compared with cells exposed to Ad-GFP control.

### Example 3: BAG3 over-expression ameliorates changes in markers of autophagy and apoptosis

NMVCs were prepared, exposed to H/R, harvested and then immunobloted as described in Example 1 above. Infection of NMVCs with Ad-BAG3 three days before evaluation modestly increased BAG3 levels (p< 0.01) when compared with NMVC's infected with Ad-GFP as shown in Figures 2A and 2B. Similarly, Ad-BAG3 substantially increased BAG3 levels in myocytes that were exposed to H/R (p<0.05) as shown in Figures 2A and 2B. Ad-BAG3 had no effect on JNK activation or on levels of cleaved caspase-3, Bcl2 and LAMP-2 in NMVCs incubated under normal conditions, as shown in Figures 2A and 2C to 2F. By contrast, NMVCs that received Ad-BAG3 3 days before H/R had significantly lower levels of p-JNK (p<0.05) and cleaved caspase-3 (p<0.05) and increased levels of Bcl2 (p<0.05) and LAMP-2 (p<0.01) when compared to control NMVCs that were infected with Ad-GFP, as shown in Figures 2A and 2C to 2F.

### Example 4: BAG3 modulates cardiomyocyte autophagy

To determine whether the changes in markers of autophagy represented an actual change in the amount of autophagy after H/R, NMVCs in which BAG3 levels were manipulated with Ad-BAG3 or Ad-siBAG3 were transfected with the double-labeled RFP-GFP-LC3-I autophagy reporter system and then exposed to H/R as described in Example 1 above. This system takes advantage of the fact that LC3-I is post-translationally modified by a ubiquitin-like system that converts it to its lapidated LC3-II form. LC3-II is sequestered into autolysosomes where it is degraded or recycled. LC3 puncta fluoresce both green and red in autophagasomes. However, in the acidic milieu of the autolysosome, the GFP fluorescence is quenched leaving predominantly red puncta. Thus, yellow puncta represent the combined fluorescence of GFP (green) and RFP (red) and reflect the presence of autophagasomes whereas red puncta represent RFP alone. In normal phagosome-lysosome fusions, there will be more red fluorescence than yellow fluorescence whereas when autophagy is impeded with diminished phagasome-lysosome fusion, yellow fluorescence is predominant. As shown in the confocal images in Figure 3A, yellow fluorescence was more prominent in NMVCs that had undergone H/R or that had been infected with siBAG3. By contrast, RFP signals were more prominent suggesting increased incorporation of LC3 into autolysosmes. The subjective evaluations of the confocal images were confirmed by counting the number of yellow and red puncta in each group (control, H/R, siBAG3 and H/R + Ad-BAG3: Figure 3B). In addition, the ratio of autolysosomes (red puncta)/autophagasomes (yellow puncta)/number of cells counted was significantly reduced after H/R, a change that was blunted by over-expression of BAG3 by Ad-BAG3 suggesting that both H/R and decreased levels of BAG3 decreased the amount of autophagy whereas BAG3 over-expression restored control levels of autophagy (Figure 3C).

### Example 5: BAG3 translocates to the peri-nuclear and nuclear region during the stress of hypoxic/re-oxygenation

Under normal conditions, confocal imaging demonstrated that BAG3 was found predominantly in the cytoplasm of neonatal mycoytes consistent with our previous observations. However, when NMVCs were exposed to H/R, BAG3 was found predominantly in the peri-nuclear region and in the nucleus as shown in Figure 4A. Knocking down BAG3 by siRNA in normoxic NMVCs also resulted in the translocation of BAG3 to the peri-nuclear region and the nucleus as shown in Figure 4A. Cell fractionation studies confirmed the morphological findings by confocal microscopy as BAG3 in the cytosolic fraction was decreased but BAG3 in the nuclear fraction was increased after H/R or after BAG3 was knocked down with siRNA (Figure 4B and 4C) As seen in Figure 4B, the specificity of the fractions was confirmed by the presence of beta-tubulin predominantly in the cytosolic extract and histone predominantly in the nuclear fraction.

### Example 6: BAG3 overexpression enhanced left ventricular function and reduced infarct size after ischemia/reperfusion (I/R) in mice

To assess whether the studies of BAG3 in NMVCs were relevant to mice *in vivo,* we measured ventricular function and infarct size after I/R in hearts in which BAG3 was over-expressed after retro-orbital injection of rAAV9 expressing myc⁻-tagged BAG3 under the control of a CMV promoter. As seen in Figure 5A and 5B, left ventricular (LV) ejection fraction (EF) measured two days after I/R in mice that had received a retro-orbital injection of rAAV9-BAG3 was significantly greater than in mice that received rAAV9-GFP control (p<0.01). Consistent with the results in the neonatal myocytes, myocardial BAG3 levels were reduced after I/R but were enhanced after rAAV9-BAG3. (Figure 5C) The injection of rAAV9-BAG3 did not change the area at risk (Figure 5D and 5E) but significantly (p<0.01) reduced infarct size at 72 hours after I/R as compared with infarct size in mice that had received an injection of rAAV9-GFP (Figure 5D and 5F).

### Example 7: BAG3 over-expression in the infarct border zone of mice after I/R recapitulated changes in markers of autophagy and apoptosis seen in NMVCs after H/R

That rAAV9-BAG3 was expressed in the mouse heart after retro-orbital injection was seen by the finding that myc⁻ expression was observed in the hearts of mice that received rAAV9-BAG3 but not in hearts of mice hat received rAAV9-GFP (Figure 6A). Consistent with the results in NMVCs, rAAV9-BAG3 significantly increased levels of Bcl2 (p<0.01; Figure 6A and 6B) and LAMP-2 (p<0.01; Figure 6A and 6B) and decreased levels of cleaved caspase-3 (p<0.01; Figure 6A and 6C) and p-JNK (p<0.01; Figure 6A and 6D).

LAMP2 is an important determinant of autophagasome-lysosome fusion, Bcl2 stimulates autophagy by disrupting its association with Beclin 1 leading to the activation of the Beclin 1-associated class III ptdlns3K complex while also playing a role in limiting apoptosis when bound to the Bcl2 binding site of BAG3, and cleaved caspase-3 is a protease responsible for chromatin margination, DNA fragmentation and nuclear collapse during the execution phase of apoptosis. However, considerable controversy has surrounded the use of biomarkers for measuring autophagy because it is a dynamic multi-step process that begins with the formation of a phagaphore, proceeds through the maturation of the phagaphore as it recruits membranes from different intracellular sources and accumulates targeted proteins, and finally it fuses with lysosomes to form an autolysosome in order to begin the process of protein digestion.

To better assess the effects of both diminished and enhanced levels of BAG3 on autophagy, we used an autophagy reporter system consisting of double labeled microtubule-associated protein light chain 3 (LC3-I). This system takes advantage of the fact that LC3-I is post-translationally modified by a ubiquitin-like system that converts it to its lapidated LC3-II form which is anchored to the outer and inner membranes of autophagasomes. LC3-II is sequestered into autolysosomes where it is degraded or recycled. The assay takes advantage of the fact that LC3 puncta fluoresce both green and red in autophagasomes. However, in the acidic milieu of the autolysosome, the GFP fluorescence is quenched leaving predominantly red puncta. These studies demonstrated that both H/R and BAG3 knockdown resulted in decreased autophagy whereas BAG3 over-expression restored the autophagy process. These results are consistent with an earlier report by Ma et al demonstrating that ischemia/reperfusion injury impairs autophagasome clearance mediated in part by reactive oxygen species-induced decline in LAMP-2.

JNK was activated (p-JNK) when BAG3 levels decreased during H/R or I/R but the level of activation decreased when BAG3 levels were increased by Ad-BAG3 or rAAV9-BAG3 in NMVCs or the adult heart respectively. JNK belongs to the MAPK family of kinases but is differentiated from other kinases in that it belongs to the group of MAPKs (p-JNK, ERK1/2, and p38) that can phosphorylate non-kinase substrates including transcription factors and scaffolding proteins. Previous studies have demonstrated that JNK is activated in the heart during reperfusion following ischemia but not by ischemia alone. Furthermore, studies in non-myocytes suggest that activation of JNK enhanced BAG3 gene expression whereas JNK inhibitors decreased BAG3 expression. Therefore, there may be a feedback loop that decreases JNK activation in the heart when BAG3 levels are high and increases JNK activation when BAG3 levels are low. However, the interaction between BAG3 and JNK is highly complex and, further studies are required to clarify the relationship between BAG3 and JNK in the heart.

BAG3 trans-located from the cytoplasm to the nucleus during the stress of hypoxia and re-oxygenation. BAG3 translocation has not been reported in myocytes. This finding is consistent with a previous study demonstrating that BAG3 can be found in the nucleus of human glial cells resulting in its ability to stimulate its own transcription through a positive feedback loop involving its 5'-untranslated sequence. Thus, in addition to the increasing list of functions for BAG3 in the heart, it appears that BAG3 can also regulate gene expression. This plasticity is due to the presence of numerous protein binding motifs within the BAG3 protein.

### Example 8: Bortezomib increased the levels of BAG3 in NMVCs

NMVCs were treated with bortezomib for 0.5, 1, 2, 4 or 18 hours after H/R. Levels of BAG3 were analyzed by immunoblotting as described in Example 1. Bortezomib treatment resulted in a time-dependent increase in levels of BAG3 relative to the vehicle treated control cells.

## Claims

1. A pharmaceutical composition comprising a nucleic acid encoding a BAG3 polypeptide or biologically active fragment thereof, or a BAG3 polypeptide or biologically active fragment thereof for use in treating or preventing ischemia/reperfusion injury of the heart in a subject, wherein the composition is for administration in a therapeutically effective amount to increase levels of BAG3 in the heart of the subject.

2. The composition for the use of claim 1, wherein the subject has been identified as being at risk of having ischemia/reperfusion injury.

3. The composition for the use of claim 1, wherein the subject has been identified as having ischemia/reperfusion injury.

4. The composition for the use of claim 2 or claim 3, wherein the ischemia/reperfusion injury is the result of myocardial infarction, atherosclerosis, peripheral vascular disorder, a pulmonary embolus, a venous thrombosis, unstable angina, stroke, endarterectomy, or aneurysm repair surgery.

5. The composition for the use of claim 1, wherein the composition is for use in preventing ischemia/reperfusion injury.

6. The composition for the use of claim 1 or claim 2, wherein the composition is administered prior to reperfusion or during reperfusion.

7. The composition for the use of claim 1 or claim 2, wherein the composition is administered intravenously.

8. The composition for the use of claim 1 or claim 2, wherein the composition is administered with a further therapeutic agent comprising: an anti-inflammatory agent, a vasodilator, a beta blocker, a cholesterol-lowering agent, a calcium channel blocker, an angiotensin-converting enzyme inhibitor or an anticoagulant.

9. The composition for the use of claim 2, wherein the subject is scheduled for a vascular interventional procedure.

10. The composition for the use of claim 9, wherein the vascular interventional or medical procedure comprises a procedure using a catheter or a stent, angioplasty or coronary artery bypass graft.

11. The composition for the use of claim 10, wherein the medical procedure comprises a procedure using an angioplasty catheter, a laser catheter, an atherectomy catheter, an angioscopy device, a beta-or gamma-radiation catheter, an intravascular ultrasound device, a rotational atherectomy device, a radioactive balloon, a heatable wire, a heatable balloon, a biodegradable stent strut, or a biodegradable sleeve.

12. The composition for the use of claim 1 or claim 2, wherein the nucleic acid encoding said BAG3 polypeptide or biologically active fragment thereof is contained in a vector.

13. The composition for the use of claim 12, wherein the vector is a recombinant viral vector.

14. The composition for the use of claim 12, wherein the recombinant viral vector comprises an adeno-associated virus (AAV) vector, a lentivirus vector, retrovirus vector or adenoviral vector.

15. The composition for the use of claim 14, wherein the AAV vector has cardiac tropism.

16. The composition for the use of claim 14, wherein the AAV vector comprises an AAV9 capsid serotype.

17. The composition for the use of claim 1 or claim 2, wherein the ischemia/reperfusion injury is the result of cardiac surgery.

## Patentansprüche

1. Pharmazeutische Zusammensetzung, umfassend eine Nukleinsäure, die für ein BAG3-Polypeptid oder ein biologisch aktives Fragment davon kodiert, oder ein BAG3-Polypeptid oder ein biologisch aktives Fragment davon, zur Verwendung beim Behandeln oder Vorbeugen von Ischämie-/Reperfusionsschäden des Herzens bei einem Subjekt, wobei die Zusammensetzung zur Verabreichung in einer therapeutisch wirksamen Menge ist, um Spiegel von BAG3 in dem Herzen des Subjekts zu erhöhen.

2. Zusammensetzung für die Verwendung nach Anspruch 1, wobei bei dem Subjekt das Risiko identifiziert worden ist, Ischämie-/Reperfusionsschäden aufzuweisen.

3. Zusammensetzung für die Verwendung nach Anspruch 1, wobei das Subjekt identifiziert worden sind, Ischämie/Reperfusionsschäden aufzuweisen.

4. Zusammensetzung für die Verwendung nach Anspruch 2 oder Anspruch 3, wobei die Ischämie-/Reperfusionsschäden das Ergebnis von Myokardinfarkt, Atherosklerose, peripherer Gefäßstörung, einer Lungenembolie, einer venösen Thrombose, instabiler Angina, Schlaganfall, Endarteriektomie oder Aneurysma-Reparaturoperation sind.

5. Zusammensetzung für die Verwendung nach Anspruch 1, wobei die Zusammensetzung zur Verwendung beim Vorbeugen von Ischämie-/Reperfusionsschäden ist.

6. Zusammensetzung für die Verwendung nach Anspruch 1 oder Anspruch 2, wobei die Zusammensetzung vor Reperfusion oder während Reperfusion verabreicht wird.

7. Zusammensetzung für die Verwendung nach Anspruch 1 oder Anspruch 2, wobei die Zusammensetzung intravenös verabreicht wird.

8. Zusammensetzung für die Verwendung nach Anspruch 1 oder Anspruch 2, wobei die Zusammensetzung mit einem weiteren therapeutischen Mittel verabreicht wird, umfassend: ein entzündungshemmendes Mittel, einen Vasodilatator, einen Betablocker, ein cholesterinsenkendes Mittel, einen Calciumkanalblocker, einen Angiotensin-umwandelnden Enzym-Inhibitor oder ein Antikoagulans.

9. Zusammensetzung für die Verwendung nach Anspruch 2, wobei für das Subjekt ein vaskulärer interventioneller Eingriff geplant ist.

10. Zusammensetzung für die Verwendung nach Anspruch 9, wobei der vaskuläre interventionelle oder medizinische Eingriff einen Eingriff unter Verwendung eines Katheters oder eines Stents, von Angioplastie oder Koronararterien-Bypass-Transplantat umfasst.

11. Zusammensetzung für die Verwendung nach Anspruch 10, wobei der medizinische Eingriff einen Eingriff unter Verwendung eines Angioplastiekatheters, eines Laserkatheters, eines Atherektomiekatheters, einer Angioskopievorrichtung, eines Beta- oder Gamma-Strahlungskatheters, einer intravaskulären Ultraschallvorrichtung, einer rotierenden Atherektomievorrichtung, eines radioaktiven Ballons, eines erhitzbaren Drahtes, eines erhitzbaren Ballons, einer biologisch abbaubaren Stentstrebe oder einer biologisch abbaubaren Hülse umfasst.

12. Zusammensetzung für die Verwendung nach Anspruch 1 oder Anspruch 2, wobei die Nukleinsäure, die für das BAG3-Polypeptid oder ein biologisch aktives Fragment davon kodiert, in einem Vektor enthalten ist.

13. Zusammensetzung für die Verwendung nach Anspruch 12, wobei der Vektor ein rekombinanter viraler Vektor ist.

14. Zusammensetzung für die Verwendung nach Anspruch 12, wobei der rekombinante virale Vektor einen Adeno-assoziierten-Virus(AAV)-Vektor, einen Lentivirus-Vektor, Retrovirus-Vektor oder Adenovirus-Vektor umfasst.

15. Zusammensetzung für die Verwendung nach Anspruch 14, wobei der AAV-Vektor kardiologischen Tropismus aufweist.

16. Zusammensetzung für die Verwendung nach Anspruch 14, wobei der AAV-Vektor einen AAV9-Kapsid-Serotyp umfasst.

17. Zusammensetzung für die Verwendung nach Anspruch 1 oder Anspruch 2, wobei die Ischämie/Reperfusionsschäden das Ergebnis von kardiologischer Operation sind.

## Revendications

1. Composition pharmaceutique comprenant un acide nucléique codant pour un polypeptide BAG3 ou un fragment biologiquement actif de celui-ci, ou un polypeptide BAG3 ou un fragment biologiquement actif de celui-ci, destinée à être utilisée dans le traitement ou la prévention d'une lésion d'ischémie/reperfusion du cœur chez un sujet, dans laquelle la composition est destinée à être administrée en une quantité thérapeutiquement efficace pour augmenter les niveaux de BAG3 dans le cœur du sujet.

2. Composition destinée à être utilisée selon la revendication 1, dans laquelle le sujet a été identifié comme présentant un risque de lésion d'ischémie/reperfusion.

3. Composition destinée à être utilisée selon la revendication 1, dans laquelle le sujet a été identifié comme présentant une lésion d'ischémie/reperfusion.

4. Composition destinée à être utilisée selon la revendication 2 ou la revendication 3, dans laquelle la lésion d'ischémie/reperfusion est le résultat d'un infarctus du myocarde, d'une athérosclérose, d'un trouble vasculaire périphérique, d'une embolie pulmonaire, d'une thrombose veineuse, d'un angor instable, d'un accident vasculaire cérébral, d'une endartériectomie ou d'une intervention chirurgicale de réparation d'un anévrisme.

5. Composition destinée à être utilisée selon la revendication 1, dans laquelle la composition est destinée à être utilisée dans la prévention d'une lésion d'ischémie/reperfusion.

6. Composition destinée à être utilisée selon la revendication 1 ou la revendication 2, dans laquelle la composition est administrée avant la reperfusion ou pendant la reperfusion.

7. Composition destinée à être utilisée selon la revendication 1 ou la revendication 2, dans laquelle la composition est administrée par voie intraveineuse.

8. Composition destinée à être utilisée selon la revendication 1 ou la revendication 2, dans laquelle la composition est administrée avec un agent thérapeutique supplémentaire comprenant : un agent anti-inflammatoire, un vasodilatateur, un bêta-bloquant, un agent hypocholestérolémiant, un bloqueur des canaux calciques, un inhibiteur de l'enzyme de conversion de l'angiotensine ou un anticoagulant.

9. Composition destinée à être utilisée selon la revendication 2, dans laquelle le sujet doit subir une intervention vasculaire.

10. Composition destinée à être utilisée selon la revendication 9, dans laquelle l'intervention vasculaire ou l'intervention médicale comprend une intervention utilisant un cathéter ou un stent, une angioplastie ou un pontage coronarien.

11. Composition destinée à être utilisée selon la revendication 10, dans laquelle l'intervention médicale comprend une intervention utilisant un cathéter d'angioplastie, un cathéter laser, un cathéter d'athérectomie, un dispositif d'angioscopie, un cathéter de rayonnement bêta ou gamma, un dispositif à ultrasons intravasculaire, un dispositif d'athérectomie rotative, un ballonnet radioactif, un fil chauffant, un ballonnet chauffant, une entretoise de stent biodégradable ou un manchon biodégradable.

12. Composition destinée à être utilisée selon la revendication 1 ou la revendication 2, dans laquelle l'acide nucléique codant pour ledit polypeptide BAG3 ou ledit fragment biologiquement actif de celui-ci est contenu dans un vecteur.

13. Composition destinée à être utilisée selon la revendication 12, dans laquelle le vecteur est un vecteur viral recombinant.

14. Composition destinée à être utilisée selon la revendication 12, dans laquelle le vecteur viral recombinant comprend un vecteur de virus adéno-associé (AAV), un vecteur lentiviral, un vecteur rétroviral ou un vecteur adénoviral.

15. Composition destinée à être utilisée selon la revendication 14, dans laquelle le vecteur AAV présente un tropisme cardiaque.

16. Composition destinée à être utilisée selon la revendication 14, dans laquelle le vecteur AAV comprend un sérotype de capside AAV9.

17. Composition destinée à être utilisée selon la revendication 1 ou la revendication 2, dans laquelle la lésion d'ischémie/reperfusion est le résultat d'une intervention chirurgicale cardiaque.
